# EUROPEAN PATENT APPLICATION

(11) **EP 3 744 335 A1**
(43) Date of publication of application: **02.12.2020**
(21) Application number: 18902274.2
(22) Date of filing: 19.10.2018
(51) Int. Cl.: A61K 33/42, A61K 9/14, A61K 9/70, A61K 33/00, A61K 33/24, A61K 33/34, A61K 33/38, A61K 47/02, A61P 27/14, A62B 18/02, A61M 15/00

(54) **MEDICINAL PREPARATION AND MEDICAL INSTRUMENT**

(30) Priority: 26.01.2018 JP 2018011114
(71) Applicant: Dr. C Medical Medicine Co., Ltd., Tokyo 163-1302 (JP)
(72) Inventor: OKAZAKI Narumi, Tokyo 163-1302 (JP)
(74) Representative: Wakerley, Helen Rachael
(86) International application number: PCT/JP2018/039040
(87) International publication number: WO 2019/146186

(57) **Abstract**

An object is to provide a pharmaceutical preparation for preventing or treating allergic conjunctivitis which is to be administered to the intranasal mucosa. Provided is a pharmaceutical preparation for preventing or treating allergic conjunctivitis which is to be administered to the intranasal mucosa, the pharmaceutical preparation including a composite particle containing one or more titanium oxide particles, one or more metal particles, and one or more calcium phosphate particles. Also provided is a medical device for preventing or treating allergic conjunctivitis, the medical device including an breathable mask portion covering the nasal cavity of a subject in need of prevention or treatment of allergic conjunctivitis, ear loop portions provided on the breathable mask portion, and a composite particle releasably attached to the breathable mask portion, the composite particle containing one or more titanium oxide particles, one or more metal particles, and one or more calcium phosphate particles.

## Description

### TECHNICAL FIELD

The present invention relates to a medical device for preventing or treating allergic conjunctivitis which is to be administered to the intranasal mucosa, a method for preventing or treating allergic conjunctivitis, a composite particle for use in a method for preventing or treating allergic conjunctivitis, and use of a composite particle for producing a pharmaceutical preparation for preventing or treating allergic conjunctivitis.

### BACKGROUND ART

Allergic conjunctivitis is a disease which is initiated by a reaction of the autoimmune system in the body to causative agents in the external environment, and house dust, mites, mold, and pollen, and the likes are known.

AS methods of treatment of allergic conjunctivitis, medical agent therapy, hyposensitization therapy, and the like are known.

For medical agent therapy, steroidal antiinflammatory agents, immunosuppressive agents, antihistaminic agents, and the like are used, but these medical agents may cause side effects. As the side effects of steroidal antiinflammatory agents, for example, side effects such as adrenal atrophy, dysfunction, and gastric ulcers are known, and as the side effects of immunosuppressive agentss, for example, side effects such as infectious disease are known, and as the side effects of antihistaminic agents, for example, side effects such as malaise, drowsiness, and vertigo are known.

There has recently been developed a medical agent that has not only an antihistaminic action, but also an antileukotriene action and a chemical mediator release inhibition action, and has a weak central nervous system depressant action, while being effective even in administration once a day. However, there are few medical agents that can suppress eye itching which is a cardinal symptom of allergic conjunctivitis.

In addition, since it is apparent that steroids which are frequently used for improving rhinitis symptoms have a risk to cause glaucoma, steroids are guided basically not to be used for eye itching.

Thus, there are very few medical agents that are effective for improving eye itching.

Hyposensitization therapy is a method in which an antigen related to allergy is identified and hyposensitization is induced by administrating the antigen intradermally, thereby inhibiting only the immunoreaction specific to the necessary antigen, but the therapy usually requires a few months to several years to exhibit sufficient effects. There is also a risk such as anaphylactic shock caused by direct systemic administration of an antigen responsible for allergy, and, the dose needs to be increased gradually from a small amount. In addition, hyposensitization therapy not only requires a long-term treatment but also involves a patient's discomfort due to injection.

Non-Patent Document 1 describes "earthplus" (trade name) which is manufactured and sold by Shinsyu Ceramics Co., Ltd. (Nagano, Japan) as having bactericidal actions against Staphylococcus aureus, Escherichia coli, and Pseudomonas aeruginosa. Earthplus is a powder of a composite material in which titanium oxide, silver, and hydroxyapatite are composited, and contains a composite particle containing one or more titanium oxide particles, one or more silver particles, and one or more hydroxyapatite particles. In the composite particle, one or more titanium oxide particles, one or more silver particles, and one or more hydroxyapatite particles are arranged in a three-dimensionally and randomly, and at least one silver particle is fixedly attached to at least one titanium oxide particle. Earthplus exerts a bactericidal action on the basis of the photocatalytic action of titanium oxide particles, the bactericidal action of silver particles, and the adsorption action of hydroxyapatite particles. In other words, earthplus adsorbs microorganisms by the adsorption action of hydroxyapatite particles and exerts a bactericidal action against microorganisms by the photocatalytic action of titanium oxide particles and the bactericidal action of silver particles.

The bactericidal action based on the photocatalytic action of earthplus is considered to be exerted by the following mechanism. When earthplus is irradiated with ultraviolet light (wavelength: 385 nm or less), electrons in the valence band of a titanium oxide particle are excited to the conduction band while absorbing energy for surpassing the forbidden band (band gap) and at the same time, positive holes (holes generated after release of the electrons) are generated in the valance band. The excited electrons migrate to a silver particle that is bonded to the titanium oxide particle, which impedes recombination of the excited electrons and the positive holes. The excited electrons undergo a reduction reaction on the surface of the titanium oxide particle and on the surface of the silver particle, and the positive holes undergo an oxidation reaction on the surface of the titanium oxide particle. For example, the excited electrons reduce oxygen to generate superoxide anions and the positive holes oxidize water to generate hydroxy radicals. It is considered that such active oxygen decomposes microorganisms, thereby the bactericidal action is exerted.

Patent Document 1 describes a functional body obtained by thermally spraying, onto the surface of a substrate, a composite ceramic composed of a powder mixture of an optical semiconductor ceramic (for example, titanium oxide), a metal (for example, silver) for forming an electrode, and a ceramic (for example, hydroxyapatite) having an adsorbing function, as having a bactericidal action against methicillin-resistant Staphylococcus aureus (MRSA).

Patent Document 2 describes an adherent treatment agent that contains a photocatalyst obtained by combining a semiconductor powder (for example, titanium oxide powder) with a metal powder (for example, silver powder), an adsorbing material (for example, hydroxyapatite) for adsorbing and retaining an object (for example, bacteria) to be treated by the photocatalytic action, as having bactericidal actions against a methicillin-resistant Staphylococcus aureus (MRSA) and Pseudomonas aeruginosa.

Patent Document 3 describes a mixture of a metal-ceramic-bonded particle in which a metal particle (for example, silver particle) and a ceramic particle (for example, titanium oxide particle) are bound and an adsorption material (for example, hydroxyapatite), as having bactericidal actions against Staphylococcus aureus and Escherichia coli in the absence of light. In Patent Document 3, the metal-ceramic-bonded particles are produced by subjecting the metal particles and the ceramic particles to a step selected from the group consisting of (1) a step of allowing the particles to pass through a zone maintained at an elevated temperature, (2) a step of pressurizing the particles using a ball mill, and (3) a step of simultaneously heating and pressurizing using any one of a ball mill, a high temperature roller, and a high temperature ultrasonic bonding method.

### CITATION LIST

### PATENT LITERATURE

Patent Document 1: Japanese Patent No. 2585946
Patent Document 2: Japanese Patent No. 2963657
Patent Document 3: Japanese Patent No. 5995100

### NON PATENT LITERATURE

Non-Patent Document 1: Eriko Kasuga et al., Bactericidal activities of woven cotton and nonwoven polypropylene fabrics coated with hydroxyapatite-binding silver / titanium dioxide ceramic noncomposite "Earth-plus", International Journal of Nanomedicine, 2011:6, pp.1937-1943

### SUMMARY OF INVENTION

### TECHNICAL PROBLEM

An object according to the present invention is to provide a pharmaceutical preparation for preventing or treating allergic conjunctivitis which is to be administered to the intranasal mucosa, a medical device for preventing or treating allergic conjunctivitis, a method for preventing or treating allergic conjunctivitis, a composite particle for use in a method for preventing or treating allergic conjunctivitis, and use of a composite particle for producing a pharmaceutical preparation for preventing or treating allergic conjunctivitis.

### SOLUTION TO PROBLEM

The present inventors have come to complete the present invention by finding out that composite particles containing one or more titanium oxide particles, one or more metal particles, and one or more calcium phosphate particles can be administered to the intranasal mucosa of a subject in need of prevention or treating of allergic conjunctivitis, thereby preventing or treating the allergic conjunctivitis of the subject. The composite particle exert the effect of prevention or treatment on allergic conjunctivitis in a subject's nasal cavity where light necessary for expression of a photocatalytic action is not present, and this is a surprising finding which is unforeseeable from conventional findings.

According to one aspect according to the present invention, the following pharmaceutical preparations are provided.
[A1] A pharmaceutical preparation for preventing or treating allergic conjunctivitis which is to be administered to the intranasal mucosa, the pharmaceutical preparation including a composite particle containing one or more titanium oxide particles, one or more metal particles, and one or more calcium phosphate particles.
[A2] The pharmaceutical preparation according to [A1], wherein in the composite particle, the one or more titanium oxide particles, the one or more metal particles, and the one or more calcium phosphate particles are arranged in a three-dimensionally and randomly
[A3] The pharmaceutical preparation according to [A1] or [A2], wherein at least one metal particle of the one or more metal particles is fixedly attached to at least one titanium oxide particle of the one or more titanium oxide particles.
[A4] The pharmaceutical preparation according to any one of [A1] to [A3], wherein the one or more metal particles are selected from the group consisting of a silver particle, a gold particle, a platinum particle, and a copper particle.
[A5] The pharmaceutical preparation according to any one of [A1] to [A4], wherein the one or more metal particles include a silver particle, and the one or more calcium phosphate particles include a hydroxyapatite particle.

According to another aspect according to the present invention, the following medical devices (hereinafter may be referred to as "first medical devices") are provided.
[B1] A medical device for preventing or treating allergic conjunctivitis, the medical device comprising a breathable mask portion covering the nasal cavity of a subject in need of prevention or treatment of allergic conjunctivitis, an ear loop portion provided on the breathable mask portion, and a composite particle detachably attached to the breathable mask portion, wherein the composite particle containing one or more titanium oxide particles, one or more metal particles, and one or more calcium phosphate particles.
[B2] The medical device according to [B1], wherein, in the composite particle, the one or more titanium oxide particles, the one or more metal particles, and the one or more calcium phosphate particles are arranged in a three-dimensionally and randomly.
[B3] The medical device according to [B1] or [B2], wherein at least one metal particle of the one or more metal particles is fixedly attached to at least one titanium oxide particle of the one or more titanium oxide particles.
[B4] The medical device according to any one of [B1] to [B3], wherein the one or more metal particles are selected from the group consisting of a silver particle, a gold particle, a platinum particle, and a copper particle.
[B5] The medical device according to any one of [B1] to [B4], wherein the one or more metal particles include a silver particle and the one or more calcium phosphate particles include a hydroxyapatite particle.

According to still another aspect according to the present invention, the following medical devices (hereinafter may be referred to as "second medical device") are provided.
[C1] A medical device for preventing or treating allergic conjunctivitis, which is inserted for use into the nasal cavity of a subject in need of prevention or treatment of allergic conjunctivitis, the medical device comprising a sheet portion to be inserted into the nasal cavity of the subject and a composite particle attached to the sheet portion, wherein the composite particle containing one or more titanium oxide particles, one or more metal particles, and one or more calcium phosphate particles.
[C2] The medical device according to [C1], wherein in the composite particle, the one or more titanium oxide particles, the one or more metal particles, and the one or more calcium phosphate particles are arranged in a three-dimensionally and randomly.
[C3] The medical device according to [C1] or [C2], wherein at least one metal particle of the one or more metal particles is fixedly attached to at least one titanium oxide particle of the one or more titanium oxide particles.
[C4] The medical device according to any one of [C1] to [C3], wherein the one or more metal particles are selected from the group consisting of a silver particle, a gold particle, a platinum particle, and a copper particle.
[C5] The medical device according to any one of [C1] to [C4], wherein the one or more metal particles comprise a silver particle and the one or more calcium phosphate particles comprise a hydroxyapatite particle.
[C6] The medical device according to any one of [C1] to [C5], wherein the sheet portion is breathable.
[C7] The medical device according to any one of [C1] to [C6], wherein the composite particle is detachably attached to the sheet portion.

According to still another aspect according to the present invention, the following methods are provided.
[D1] A method for preventing or treating allergic conjunctivitis, the method includes a step of administering a composite particle to the intranasal mucosa of a subject in need of prevention or treatment of allergic conjunctivitis, wherein the composite particle containing one or more titanium oxide particles, one or more metal particles, and one or more calcium phosphate particles.
[D2] The method according to [D1], wherein in the composite particle, the one or more titanium oxide particles, the one or more metal particles, and the one or more calcium phosphate particles are arranged in a three-dimensionally and randomly.
[D3] The method according to [D1] or [D2], wherein at least one metal particle of the one or more metal particles is fixedly attached to at least one titanium oxide particle of the one or more titanium oxide particles.
[D4] The method according to any one of [D1] to [D3], wherein the one or more metal particles are selected from the group consisting of a silver particle, a gold particle, a platinum particle, and a copper particle.
[D5] The method according to any one of [D1] to [D4], wherein the one or more metal particles comprise a silver particle and the one or more calcium phosphate particles comprise a hydroxyapatite particle.
[D6] The method according to any one of [D1] to [D5], wherein, in the administration step, the composite particle is administered to the intranasal mucosa of the subject by administering the pharmaceutical preparation according to the present invention (in other words, the pharmaceutical preparation according to any one of [A1] to [A5]) to the intranasal mucosa.
[D7] The method according to any one of [D1] to [D5], wherein, in the administration step, the composite particle is administered to the intranasal mucosa of the subject by putting the first medical device (in other words, the medical device according to any one of [B1] to [B5]) to the face of the subject.
[D8] The method according to any one of [D1] to [D5], wherein, in the administration step, the composite particle is administered to the intranasal mucosa of the subject by inserting the second medical device (in other words, the medical device according to any one of [C1] to [C7]) into the nasal cavity of the subject.

According to still another aspect according to the present invention, the following composite particles are provided.
[E1] A composite particle for use in a method for preventing or treating allergic conjunctivitis, the composite particle containing one or more titanium oxide particles, one or more metal particles, and one or more calcium phosphate particles.
[E2] The composite particle according to [E1], wherein in the composite particle, the one or more titanium oxide particles, the one or more metal particles and the one or more calcium phosphate particles are arranged in a three-dimensionally and randomly.
[E3] The composite particle according to [E1] or [E2], wherein at least one metal particle of the one or more metal particles is fixedly attached to at least one titanium oxide particle of the one or more titanium oxide particles.
[E4] The composite particle according to any one of [E1] to [E3], wherein the one or more metal particles are selected from the group consisting of a silver particle, a gold particle, a platinum particle, and a copper particle.
[E5] The composite particle according to any one of [E1] to [E4], wherein the one or more metal particles comprise a silver particle and the one or more calcium phosphate particles comprise a hydroxyapatite particle.
[E6] The composite particle according to any one of [E1] to [E5], wherein, in the method, the composite particle is administered to the intranasal mucosa.

According to still another aspect according to the present invention, the following use is provided.
[F1] Use of a composite particle for producing a pharmaceutical preparation for preventing or treating allergic conjunctivitis, the composite particle containing one or more titanium oxide particles, one or more metal particles, and one or more calcium phosphate particles.
[F2] The use according to [F1], wherein, in the composite particle, the one or more titanium oxide particles, the one or more metal particles, and the one or more calcium phosphate particles are arranged in a three-dimensionally and randomly.
[F3] The use according to [F1] or [F2], wherein at least one metal particle of the one or more metal particles is fixedly attached to at least one titanium oxide particle of the one or more titanium oxide particles.
[F4] The use according to any one of [F1] to [F3], wherein the one or more metal particles are selected from the group consisting of a silver particle, a gold particle, a platinum particle, and a copper particle.
[F5] The use according to any one of [F1] to [F4], wherein the one or more metal particles comprise a silver particle and the one or more calcium phosphate particles comprise a hydroxyapatite particle.

### ADVANTAGEOUS EFFECTS OF INVENTION

According to the present invention, a pharmaceutical preparation for preventing or treating allergic conjunctivitis which is to be administered to the intranasal mucosa, a medical device for preventing or treating allergic conjunctivitis, a method for preventing or treating allergic conjunctivitis, a composite particle for use in a method for preventing or treating allergic conjunctivitis, and use of a composite particle for producing a pharmaceutical preparation for preventing or treating allergic conjunctivitis are provided.

### BRIEF DESCRIPTION OF DRAWINGS

[Fig. 1] It is a partially cutaway view illustrating one embodiment of the first medical device according to the present invention.
[Fig. 2] It is a perspective view illustrating one embodiment of the second medical device according to the present invention.
[Fig. 3] It is a view illustrating a result of the electron microscope observation (500x magnification) of the composite particles-attached nonwoven fabric produced in Production Example 2.
[Fig. 4] It is a view illustrating a result of the electron microscope observation (5000 x magnification) of the composite particles-attached nonwoven fabric produced in Example 2.
[Fig. 5] It is a view illustrating a result of the electron microscope observation (20000x magnification) of the composite particles-attached nonwoven fabric produced in Example 2.
[Fig. 6] It is a view illustrating a result of the electron microscope observation (500x magnification) of the nonwoven fabric with no composite particle attached.
[Fig. 7] It is a view illustrating a result of the electron microscope observation (1000x magnification) of the nonwoven fabric with no composite particle attached.
[Fig. 8] It is a view showing distribution diagrams on the predictive value by a linear regression model of a HATS using group and a HATS non-using group with respect to the eye itching in Test Example.
[Fig. 9] It is a view showing an ROC curve on the predictive value by the linear regression model of the HATS using group and HATS non-using group with respect to the eye itching in Test Example.

### DESCRIPTION OF EMBODIMENTS

Below, the present invention will be described in detail below.

### Composite Particles

Unless otherwise specified, the following description about the composite particle applies to any aspects encompassed in the present invention.

The composite particle used in the present invention contains one or more titanium oxide particles, one or more metal particles, and one or more calcium phosphate particles.

The number of the titanium oxide particles per composite particle may be one or may be two or more. The number of the titanium oxide particles per composite particle is generally two or more.

The form of the titanium oxide particle contained in the composite particle is not particularly limited, and examples thereof include a spherical, granular, acicular, flaky, and amorphous form. The composite particle may contain two or more titanium oxide particles having different forms.

The particle size of the titanium oxide particle contained in the composite particle is not particularly limited as long as it is smaller than the particle size of the composite particle, and the particle size can be appropriately adjusted according to the particle size of the composite particle. The titanium oxide particle contained in the composite particle is, for example, a nanoparticle or a submicron particle.

Examples of crystal structures of the titanium oxide constituting the titanium oxide particle include an anatase type, a rutile type, and a brookite type, and among them, an anatase type is preferred.

The number of the metal particles per composite particle may be one or may be two or more. The number of the metal particles per composite particle is generally two or more.

The form of the metal particle contained in the composite particle is not particularly limited, and examples thereof include a spherical, granular, acicular, flaky, and amorphous form. The composite particle may contain two or more metal particles having different forms.

The particle size of the metal particle contained in the composite particle is not particularly limited as long as it is smaller than the particle size of the composite particle, and the particle size can be appropriately adjusted according to the particle size of the composite particle. The metal particle contained in the composite particle is, for example, a nanoparticle or a submicron particle.

The metal particle contained in the composite particle is, for example, selected from the group consisting of a silver particle, a gold particle, a platinum particle, and a copper particle. The metal particle contained in the composite particle is preferably a silver particle. The composite particle may contain two or more metal particles of different kinds.

The number of the calcium phosphate particles per composite particle may be one or may be two or more. The number of the calcium phosphate particles per composite particle is generally two or more.

The form of the calcium phosphate particle contained in the composite particle is not particularly limited, and examples thereof include a spherical, granular, acicular, flaky, and amorphous form. The composite particle may contain two or more calcium phosphate particles having different forms .

The particle size of the calcium phosphate particle contained in the composite particle is not particularly limited as long as it is smaller than the particle size of the composite particle, and the particle size can be appropriately adjusted according to the particle size of the composite particle. The calcium phosphate particle contained in the composite particle is, for example, a nanoparticle or a submicron particle.

Examples of calcium phosphates constituting the calcium phosphate particle include apatite, tricalcium phosphate, and octacalcium phosphate, and among them, apatite is preferred. Examples of apatites include hydroxyapatite, fluoroapatite, and carbonate apatite, and among them, hydroxyapatite (Ca₁₀(PO₄)₆(OH)₂) is preferred.

The contents of the titanium oxide particles, the metal particles, and the calcium phosphate particles in one composite particle are not particularly limited, but the lower limit of the content of the titanium oxide particles is generally 10 parts by mass, preferably 20 parts by mass, further preferably 25 parts by mass, and furthermore preferably 30 parts by mass relative to 1 part by mass of the metal particles, and the upper limit of the content of the titanium oxide particles is generally 300 parts by mass, preferably 250 parts by mass, further preferably 200 parts by mass, and furthermore preferably 180 parts by mass relative to 1 part by mass of the metal particles. In addition, the lower limit of the content of the calcium phosphate particles is generally 1 parts by mass, preferably 2 parts by mass, and further preferably 3 parts by mass relative to 1 part by mass of the metal particles, and the upper limit of the content of the calcium phosphate particles is generally 100 parts by mass, preferably 80 parts by mass, further preferably 60 parts by mass, furthermore preferably 50 parts by mass.

The particle size of the composite particle measured by dynamic light scattering is preferably 100 to 600 nm, further preferably 200 to 500 nm, and furthermore preferably 250 to 350 nm. The measurement of the particle size by dynamic light scattering is performed with a commercially available dynamic light scattering particle size distribution analyzer, preferably a dynamic light scattering Nanotrac particle size distribution analyzer "UPA-EX150" (manufactured by Nikkiso Co. Ltd.) .

In the composite particle, one or more titanium oxide particles, one or more metal particles, and one or more calcium phosphate particles are preferably arranged in a three-dimensionally and randomly.

In an embodiment of the three-dimensionally and randomly arrangement, at least one metal particle is fixedly attached to at least one titanium oxide particle.

In an embodiment of the three-dimensionally and randomly arrangement, around a certain one particle (one particle selected from a titanium oxide particle, a metal particle, and a calcium phosphate particle), other one or more particles (one or more particles selected from titanium oxide particles, metal particles, and calcium phosphate particles) exist. In this embodiment, to the certain one particle, one or more particles of the same kind may be adjacent, or one or more particles of different kind(s) may be adjacent. The adjacent particles are preferably bonded or fixedly attached to each other. Examples of combinations of adjacent particles include a titanium oxide particle and a titanium oxide particle, a metal particle and a metal particle, a calcium phosphate particle and a calcium phosphate particle, a titanium oxide particle and a metal particle, a titanium oxide particle and a calcium phosphate particle, and a metal particle and a calcium phosphate particle.

In an embodiment of the three-dimensionally and randomly arrangement, a part of at least one particle selected from a titanium oxide particle, a metal particle, and a calcium phosphate particle is exposed on the surface of the composite particle.

In an embodiment of the three-dimensionally and randomly arrangement, a part of at least one titanium oxide particle, a part of at least one metal particle, and a part of at least one calcium phosphate particle are exposed on the surface of the composite particle.

In an embodiment of the three-dimensionally and randomly arrangement, at least one particle selected from a titanium oxide particle, a metal particle, and a calcium phosphate particle is not exposed on the surface of the composite particle but exists in the interior of the composite particle.

In an embodiment of the three-dimensionally and randomly arrangement, at least one particle selected from a titanium oxide particle, a metal particle, and a calcium phosphate particle has a film form, and exists on at least a part of the surface of the composite particle.

In an embodiment of the three-dimensionally and randomly arrangement, two or more particles selected from titanium oxide particles, metal particles, and calcium phosphate particles integrally or continuously have a film form, and exist on at least a part of the surface of the composite particle.

Whether a certain one particle has a particulate form, or has a film form, or integrally or continuously has a film form together with other one or more particles may be affected by the ratio of particles blended or the like in the production of the composite particle. Depending on the blend ratio, a certain one particle may no longer maintain a particulate form, but take a film form that exists on at least a part of the surface of the composite particle. For example, when particles are composited by a mechanical technique, such as a bead mill and a ball mill, a particle (for example, a silver particle) of a material that has a lower hardness than the other particles may have such a film form.

Two or more embodiments of the above embodiments of the three-dimensionally and randomly arrangement may be combined.

As the composite particle, for example, a composite material powder sold from Shinsyu Ceramics Co., Ltd. under a trade name of "earthplus" can be used. Earthplus is a powder of a composite material in which titanium oxide, silver, and hydroxyapatite are composited, and contains a composite particle that contains one or more titanium oxide particles, one or more silver particles, and one or more hydroxyapatite particles. In the composite particle, one or more titanium oxide particles, one or more silver particles, and one or more hydroxyapatite particles are arranged in a three-dimensionally and randomly, and at least one silver particle is fixedly attached to at least one titanium oxide particle. In addition, in the composite particle, a part of at least one titanium oxide particle, a part of at least one silver particle, and a part of at least one hydroxyapatite particle are considered to be exposed on the surface of the composite particle.

The composite particle can be produced, for example, by mixing a titanium oxide powder, a metal powder, and a calcium phosphate powder in a liquid using a wet mill to composite one or more titanium oxide particles contained in the titanium oxide powder, one or more metal particles contained in the metal powder, and one or more calcium phosphate particles contained in the calcium phosphate powder. Note that the thus-produced composite particle is subsequently used in the present invention without being sintered.

The titanium oxide content in the titanium oxide powder (purity) is preferably 90% by weight or more, further preferably 95% by weight or more, and furthermore preferably 98% or more. The upper limit is, for example, 99%.

The particle size of the titanium oxide particle (primary particle) contained in the titanium oxide powder is not particularly limited, and is, for example, 0.03 to 0.1 µm. Since a wet mill can disperse an agglomerate of particles into individual particles, an agglomerate (secondary particle) of titanium oxide particles may be contained in the titanium oxide powder. The particle size of the agglomerate of titanium oxide particles is, for example, 1 to 2 µm. The particle size of the titanium oxide particle or an agglomerate of the titanium oxide particles is measured, for example, using a transmission electron microscope (TEM) or a scanning electron microscope

(SEM) .

The metal content in the metal powder (purity) is preferably 80% by weight or more, further preferably 95% by weight or more, and furthermore preferably 98% or more. The upper limit is, for example, 99.9%.

The particle size of the metal particle (primary particle) contained in the metal powder is not particularly limited, and is, for example, 1.1 to 1.9 µm. Since a wet mill can disperse an agglomerate of particles into individual particles, an agglomerate (secondary particle) of metal particles may be contained in the metal powder. Note that agglomeration of the metal particles contained in the metal powder can be suppressed by storing the metal powder in a freezer until use. The particle size of the metal particle or an agglomerate of the metal particles is calculated, for example, based on the specific surface area.

The calcium phosphate content in the calcium phosphate powder (purity) is preferably 90% by weight or more, further preferably 95% by weight or more, and furthermore preferably 98% or more.

The particle size of the calcium phosphate particle (primary particle) contained in the calcium phosphate powder is not particularly limited, and is, for example, 0.1 to 0.2 µm. Since a wet mill can disperse an agglomerate of particles into individual particles, an agglomerate (secondary particle) of calcium phosphate particles can be contained in the calcium phosphate powder. The particle size of the agglomerate of calcium phosphate particles is, for example, 4 to 5 µm. The particle size of the calcium phosphate particle or an agglomerate of the calcium phosphate particles is measured, for example, by a laser diffraction scattering method.

A wet mill can composite one or more titanium oxide particles, one or more metal particles, and one or more calcium phosphate particles while dispersing and pulverizing, in a liquid, the particles contained in the titanium oxide powder, the metal powder, and the calcium phosphate powder. Examples of wet mills include a bead mill and a ball mill, and among them, a bead mill is preferred. Examples of materials of pulverizing media, such as beads and balls, to be used in mills, such as a bead mill and a ball mill, include alumina, zircon, zirconia, steel, and glass, and among them, zirconia is preferred. The size (diameter) of the pulverizing media can be appropriately adjusted according to the particle size of the composite particle to be produced and the like, and is generally 0.05 to 3.0 mm, preferably 0.1 to 0.5 mm. As the pulverizing media, for example, beads or balls having a size of about 0.1 mm and a mass of about 0.004 mg can be used.

The liquid used in mixing is, for example, an aqueous medium, such as water. When the liquid used in mixing is water, the total amount of the titanium oxide powder, the metal powder, and the calcium phosphate powder blended is adjusted to generally 25 to 45 parts by mass, preferably 30 to 40 parts by mass relative to 65 parts by mass pf water.

When the raw materials including a titanium oxide powder, a metal powder, a calcium phosphate powder, and a liquid are mixed with a wet mill, various conditions, such as, for example, the total amount of the raw material powders added, the flow rate of the liquid, the circumferential speed of the blade in the cylinder, the stirring temperature, and the stirring time, can be appropriately adjusted according to the particle size of the composite particle to be produced and the like. The total amount of the raw material powders (the titanium oxide powder, the metal powder, and the calcium phosphate powder) added is, for example, 4 kg or more, and the volume of the cylinder is, for example, 0.5 to 4 L, the flow rate of the liquid is, for example, 0.5 to 3 L/minutes, the circumferential speed of the blade is, for example, 300 to 900 m/minute, the liquid temperature is, for example, 20 to 60°C, and the mixing time per kg of the raw material powders is, for example, 0.5 to 2 hours. The upper limit of the total amount of the raw material powders added can be appropriately adjusted according to the volume of the cylinder and the like. The mixing time can be appropriately adjusted according to the total amount of the raw material powders added and the like.

In addition to the titanium oxide powder, the metal powder, the calcium phosphate powder, and the liquid, a dispersant is preferably incorporated in the raw materials. Examples of dispersants include a high molecular weight dispersant, a low molecular weight dispersant, and an inorganic dispersant, and the dispersant can be appropriated selected according to the type of the liquid used in wet mixing. When the liquid used in mixing is an aqueous medium, such as water, for example, an anionic high molecular weight dispersant, a nonionic high molecular weight dispersant, and the like can be used as the dispersant. Examples of anionic high molecular weight dispersants include a polycarbocylic acid dispersant and a naphthalenesulfonic acid formaldehyde condensate dispersant, and an example of a nonionic high molecular weight dispersant is polyethylene glycol. The amount of the dispersant added can be appropriately adjusted, and is, for example, 0.1 to 3% by mass, preferably 0.3 to 1% by mass relative to 35 parts by mass of the total amount of the titanium oxide powder, the metal powder, and the calcium phosphate powder blended.

The titanium oxide powder, the metal powder, and the calcium phosphate powder are mixed in a liquid using a wet mill to composite one or more titanium oxide particles contained in the titanium oxide powder, one or more metal particles contained in the metal powder, and one or more calcium phosphate particles contained in the calcium phosphate powder, thereby a suspension (slurry) of the composite particles can be produced. Then, the solvent in the suspension is removed by evaporation or the like, thereby an assembly of composite particles (dry powder) can be produced. An assembly of composite particles (dry powder) can also be produced from a suspension (slurry) of the composite particles by a known granulation method, such as spray dry granulation.

The particle size of an assembly of composite particles measured by dynamic light scattering is, for example, 100 to 600 nm, preferably 200 to 500 nm. The median diameter (d50) of an assembly of composite particles measured by volume by dynamic light scattering is, for example, 250 to 350 nm, preferably about 300 nm. The particle size by dynamic light scattering is measured using a commercially available dynamic light scattering particle size distribution analyzer, preferably a dynamic light scattering Nanotrac particle size distribution analyzer "UPA-EX150" (manufactured by Nikkiso Co. Ltd.) .

The produced composite particles can be used in the present invention as it is, but the particle size thereof may be adjusted before used in the present invention. The adjustment of the particle size can be performed, for example, by sieving the composite particles in the powder state or suspension state.

In an assembly of the composite particles, the numbers of titanium oxide particles, metal particles, and calcium phosphate particles per composite particle may be the same or different among composite particles.

In addition to the composite particles containing one or more titanium oxide particles, one or more metal particles, and one or more calcium phosphate particles, other particles that may be produced as byproducts in production of the composite particles may coexist in the assembly of the composite particles. Examples of such other particles include an independent titanium oxide particle, an independent metal particle, an independent calcium phosphate particle, a bonded body of titanium oxide particles (containing no metal particle and no calcium phosphate particle), a bonded body of metal particles (containing no titanium oxide particle and no calcium phosphate particle), a bonded body of calcium phosphate particles (containing no titanium oxide particle and no metal particle), a bonded body of a titanium oxide particle and a metal particle (containing no calcium phosphate particle), a bonded body of a titanium oxide particle and a calcium phosphate particle (containing no metal particle), and a bonded body of a metal particle and a calcium phosphate particle (containing no titanium oxide particle).

The composite particle can be used in a method for preventing or treating allergic conjunctivitis as described later. Accordingly, the present invention also encompasses a composite particle for use in a method for preventing or treating allergic conjunctivitis.

The composite particle can be used for producing a pharmaceutical preparation for preventing or treating allergic conjunctivitis as described later. Accordingly, the present invention also encompasses use of a composite particle for producing a pharmaceutical preparation for preventing or treating allergic conjunctivitis.

### Pharmaceutical preparation

The pharmaceutical preparation according to the present invention is a pharmaceutical preparation which is to be administered to the intranasal mucosa, and contains a composite particle containing one or more titanium oxide particles, one or more calcium phosphate particles, and one or more metal particles. The foregoing description applies to the composite particle.

The pharmaceutical preparation according to the present invention can be administered to the intranasal mucosa of a subject in need of prevention or treatment of allergic conjunctivitis, thereby preventing or treating the allergic conjunctivitis of subject. The prevention of allergic conjunctivitis includes preventing symptoms such as eye itching, hyperemia, foreign body sensation, eye discharge, and easy-to-appear tears which may occur in a subject in the future, and the treatment of allergic conjunctivitis includes improving or inhibiting symptoms such as eye itching, hyperemia, foreign body sensation, eye discharge, and easy-to-appear tears which has already occurred in a subject.

Allergic conjunctivitis to which the pharmaceutical preparation according to the present invention is applied is not limited to a particular symptom as long as they has inflammation occurring to the conjunctiva and has a symptom, such as eye itching, hyperemia, foreign body sensation, eye discharge, or easy-to-appear tears. The pharmaceutical preparation according to the present invention can be applied to various allergic conjunctivitis.

Allergic conjunctivitis to which the pharmaceutical preparation according to the present invention is applied can be classified into perennial allergic conjunctivitis and seasonal allergic conjunctivitis in accordance with the predilection time. Allergic conjunctivitis to which the pharmaceutical preparation according to the present invention is applied may be perennial allergic conjunctivitis or seasonal allergic conjunctivitis. The pharmaceutical preparation according to the present invention can exert an excellent prevention or treatment effect on allergic conjunctivitis which has been considered difficult to prevent or treat, particular perennial allergic conjunctivitis which is initiated by house dust, mites, mold, or the like or on seasonal allergic conjunctivitis which is initiated by pollen of a plant, such as Japanese cedar, Japanese cypress, ragweed, Dactylis glomerata, Alnus, or wormwood.

Allergic conjunctivitis generally includes a combination-type allergic conjunctivitis in which two or more of symptoms, such as eye itching, hyperemia, foreign body sensation, eye discharge, and easy-to-appear tears, for example, eye itching and hyperemia, or eye itching and foreign body sensation, are combined.Allergic conjunctivitis is initiated by the reaction of the immune system in the body to causative agents in the external environment. Examples of causative agents for allergic conjunctivitis include, for example, house dust, mites, mold, pollen, grass, trees, animals and the like. More specifically, the allergic conjunctivitis is a type I allergic disease of the conjunctiva, and is characterized by paroxysmal and recurrent symptoms, such as eye itching, hyperemia, foreign body sensation, eye discharge, and easy-to-appear tears, in principle.
Because allergic conjunctivitis is a type I allergic disease, a patient which allergic conjunctivitis may have an allergic predisposition (anamnesis, complication, and family history of allergy), and may sometimes has characteristics of an increased serum-specific IgE antibody level, increased local mast cells and eosinophils, nonspecific hypersensitivity of mucosa, and the like. Among the types of the allergic conjunctivitis, perennial allergic conjunctivitis is often initiated by house dust or mites and the seasonal allergic conjunctivitis is often initiated by pollen.

The subject to whom the pharmaceutical preparation according to the present invention is to be administered is not particularly limited as long as the subject needs to prevent or treat allergic conjunctivitis, but is generally an allergic conjunctivitis patient.

The dose of the pharmaceutical preparation according to the present invention is an amount effective for prevention or treatment of the allergic conjunctivitis.

The amount effective for prevention and treatment of the allergic conjunctivitis can be appropriately adjusted according to the dosage form of the pharmaceutical preparation, the degree of the allergic conjunctivitis, the dosage regimen, and the like. The dose of the pharmaceutical preparation according to the present invention per administration is adjusted so as to provide an amount of the composite particles administered per administration of generally 0.1 to 10 µg, preferably 0.2 to 5 µg, and further preferably 0.4 to 4 µg. The number of administrations of the pharmaceutical preparation according to the present invention per day is not particularly limited, but generally is once to 5 times, preferably once to 3 times, and further preferably once to twice. The interval between administrations of the pharmaceutical preparation according to the present invention can be appropriately adjusted in view of, for example, the duration of the effect of prevention or treatment of allergic conjunctivitis. The duration of the effect of prevention or treatment of allergic conjunctivitis that is expected when the pharmaceutical preparation according to the present invention is administered to a subject for one day in the above dose per administration at the above number of administrations per day, although may vary among individual subjects, is generally several hours to several days. The duration of the effect of prevention or treatment of allergic conjunctivitis that is expected when the pharmaceutical preparation according to the present invention is administered to a subject for one to two weeks in the above dose per administration at the above number of administrations per day, although may vary among individual subjects, is generally one week to two months.

In addition to the composite particle which is an active ingredient, any pharmaceutically acceptable additive can be blended to produce the pharmaceutical preparation according to the present invention. Examples of such additives include a pH modifier, a preservative, a flavoring agent, a dispersant, a humectant, a stabilizer, an antiseptic, a suspending agent, and a surfactant.

As a pH modifier, one appropriately selected from pH modifiers that are generally used in external preparations can be used. The amount of the pH modifier blended can be appropriately adjusted according to the dosage form, the components of the base, and the like. Examples of pH modifiers include inorganic acids, such as hydrochloric acid, sulfuric acid, nitric acid, hydrobromic acid, and phosphoric acid, organic acids, such as acetic acid, succinic acid, fumaric acid, malic acid, oxalic acid, lactic acid, glutaric acid, salicylic acid, and tartaric acid, and salts of these acids. One pH modifier may be used alone or two or more pH modifiers may be used in combination.

As a preservative, one appropriately selected from preservatives that are generally used in external preparations can be used. The amount of the preservative blended can be appropriately adjusted according to the dosage form, the components of the base, and the like. Examples of preservatives include p-hydroxybenzoic acid, methylparaben, ethylparaben, propylparaben, chlorobutanol, benzyl alcohol, and p-hydroxybenzoic acid alkyl esters, such as methyl p-hydroxybenzoate and propyl p-hydroxybenzoate. One preservative may be used alone or two or more preservatives may be used in combination.

As a flavoring agent, one appropriately selected from flavoring agents that are generally used in external preparations can be used. The amount of the flavoring agent blended can be appropriately adjusted according to the dosage form, the components of the base, and the like. Examples of flavoring agents include menthol, rose oil, eucalyptus oil, and d-camphor. One flavoring agent may be used alone or two or more flavoring agents may be used in combination.

As a dispersant, one appropriately selected from dispersants that are generally used in external preparations can be used. The amount of the dispersant blended can be appropriately adjusted according to the dosage form, the components of the base, and the like. Examples of dispersants include sodium metaphosphate, calcium polyphosphate, and silicic anhydride. One dispersant may be used alone or two or more dispersants may be used in combination.

As a humectant, one appropriately selected from humectants that are generally used in external preparations can be used. The amount of the humectant blended can be appropriately adjusted according to the dosage form, the components of the base, and the like. Examples of humectants include propylene glycol, butylene glycol, glycerol, sorbitol, sodium lactate, and sodium hyaluronate. One humectant may be used alone or two or more humectants may be used in combination.

As a stabilizer, one appropriately selected from stabilizers that are generally used in external preparations can be used. The amount of the stabilizer blended can be appropriately adjusted according to the dosage form, the components of the base, and the like. Examples of stabilizers include sodium hydrogen sulfite, tocopherol, ethylenediaminetetraacetic acid (EDTA), and citric acid. One stabilizer may be used alone or two or more stabilizers may be used in combination.

As an antiseptic, one appropriately selected from antiseptics that are generally used in external preparations can be used. The amount of the antiseptic blended can be appropriately adjusted according to the dosage form, the components of the base, and the like. Examples of antiseptics include ethyl p-hydroxybenzoate, propyl p-hydroxybenzoate, benzalkonium hydrochloride, and sorbic acid. One antiseptic may be used alone or two or more antiseptics may be used in combination.

As a suspending agent, one appropriately selected from suspending agents that are generally used in external preparations can be used. The amount of the suspending agent blended can be appropriately adjusted according to the dosage form, the components of the base, and the like. Examples of suspending agents include powdered tragacanth, powdered gum arabic, bentonite, and sodium carboxymethylcellulose. One suspending agent may be used alone or two or more suspending agents may be used in combination.

As a surfactant, one appropriately selected from surfactants that are generally used in external preparations can be used. The amount of the surfactant blended can be appropriately adjusted according to the dosage form, the components of the base, and the like. Examples of surfactants include polyoxyethylene hydrogenated castor oil, sorbitan fatty acid ester, such as sorbitan sesquioleate, and polyoxyl stearate. One surfactant may be used alone or two or more surfactants may be used in combination.

The dosage form of the pharmaceutical preparation according to the present invention is not particularly limited as long as the pharmaceutical preparation can be administered to the intranasal mucosa, and examples thereof include nasal drops, spray, aerosol, ointment, cream, lotion, liniment, cataplasm, plaster, patch, emplastrum, gel, liquid, tape, powder, and granules. The pharmaceutical preparation can be formed into a desired dosage form according to a common method described in the General Rules for Preparations issued from the Japanese Pharmacopeia, or the like using additives, a base, and the like suitable for the dosage form. Examples of the substrates to be used in administration dosage forms or patch, tape, and the like include woven fabrics, such as cotton, staple fibers, hemp, and chemical fibers; nonwovens fabric, such as rayon, polyester, and nylon; and plastic films, such as soft polyvinyl chloride, polyethylene, and polyurethane. The substrate can be a laminate sheet composed of two or more layers.

When the dosage form is ointment or cream, an oleaginous base or an emulsion base can be used as a base.

Examples of oleaginous bases include hydrocarbon, higher alcohols, higher fatty acids, higher fatty acid esters, glycols, vegetable oils, and animal oils. One oleaginous base may be used alone or two or more oleaginous bases may be used in combination.

Examples of hydrocarbons usable as an oleaginous base include hydrocarbons having 12 to 32 carbon atoms, mixtures of various hydrocarbons, such as liquid paraffin, branched paraffin, solid paraffin, white petrolatum, and yellow petrolatum, squalene, squalane, and Plastibase.

Examples of higher alcohols usable as an oleaginous base include aliphatic monohydric alcohols having 12 to 30 carbon atoms, such as lauryl alcohol, tridecyl alcohol, myristyl alcohol, pentadecyl alcohol, cetyl alcohol (cetanol), hexadecyl alcohol, heptadecyl alcohol, stearyl alcohol, oleyl alcohol, nonadecyl alcohol, eicosyl alcohol, ceryl alcohol, mellissyl alcohol, and cetostearyl alcohol.

Examples of higher fatty acids usable as an oleaginous base include saturated or unsaturated fatty acids having 6 to 32 carbon atoms, such as caproic acid, enanthic acid, caprylic acid, pelargonic acid, capric acid, undecylic acid, lauric acid, tridecylic acid, myristic acid, pentadecylic acid, palmitic acid, heptadecylic acid, stearic acid, oleic acid, nonadecanoic acid, arachic acid, arachidonic acid, linoleic acid, linolenic acid, behenic acid, lignoceric acid, cerotic acid, heptacosanoic acid, montanic acid, melissic acid, lacceric acid, elaidic acid, and brassidic acid.

Examples of higher fatty acid esters usable as an oleaginous base include: fatty acid esters, such as myristyl palmitate, stearyl stearate, myristyl myristate, ceryl lignocerate, lacceryl cerotate, and lacceryl laccerate; esters of a fatty acid having 10 to 32 carbon atoms and an aliphatic monohydric alcohol having 14 to 32 carbon atoms, for example, animal-derived natural waxes, such as lanolin, bees wax, spermaceti, and shellac wax, and plant-derived natural waxes, such as carnauba wax and candelilla wax; and esters of saturated or unsaturated fatty acid having 10 to 22 carbon atoms and glycerol, such as glyceryl monolaurate, glyceryl monomyristate, glyceryl monooleate, glyceryl monostearate, glyceryl dilaurate, glyceryl dimyristate, glyceryl distearate, glyceryl trilaurate, glyceryl trimyristate, and glyceryl tristearate, or hydrogenated products thereof.

Examples of glycols usable as an oleaginous base include ethylene glycol, diethylene glycol, propylene glycol, 1,3-butanediol, and polyethylene glycol.

Examples of vegetable oils usable as an oleaginous base include camellia oil, castor oil, olive oil, cacao oil, coconut oil, palm oil, macadamia nut oil, soybean oil, Camellia sinensis seed oil, sesame oil, almond oil, safflower oil, cottonseed oil, turpentine oil, and vegetable oils and fats obtained by hydrogenating these vegetable oils.

Examples of animal oils usable as an oleaginous base include mink oil, egg-yolk oil, squalane, squalene, lanolin, and derivatives of animal oils.

Examples of emulsion bases include an oil-in-water base, a water-in-oil base, and a suspension base. One emulsion base may be used alone or two or more emulsion bases may be used in combination.

An example of an oil-in-water base is a base in which a component, such as lanolin, propylene glycol, stearyl alcohol, petroleum, silicone oil, liquid paraffin, glyceryl monostearate, or polyethylene glycol, are emulsified and dispersed in an aqueous phase in the presence or absence of a surfactant. An oil-in-water base can be suitably used in preparing a cream or the like.

An example of a water-in-oil base is a base in which water is added to a component, such as petroleum, a higher aliphatic alcohol, or liquid paraffin, in the presence of a nonionic surfactant and the components were emulsified and dispersed.

An oil-in-water base and a water-in-oil base can be used in a dosage form containing water, for example, in a liquid, a lotion, a cataplasm, or an ointment which contains water.

An example of a suspension base is an aqueous base in which a suspending agent, such as starch, glycerol, high viscosity carboxymethylcellulose, or carboxyvinyl polymer, was added to water to form a gel.

The pharmaceutical preparation according to the present invention can be produced according to a commonly adopted preparation method of an external preparation. For example, an ointment or a cream can be produced by kneading, emulsifying, and suspending raw materials of a base according to each dosage form to prepare a base, and then adding an active ingredient and various additives thereto and mixing the mixture. In mixing, a commonly used mixer, such as a screw mixer, a homomixer, a kneader, or a roll mill, can be used.

When the dosage form is lotion, it may be any of a suspension type, an emulsion type, and a solution type.

Examples of bases of a suspension type lotion include a mixture of water and a suspending agent, for example, a gum, such as gum arabic or tragacanth gum, a cellulose, such as methylcellulose, hydroxyethyl cellulose, or hydroxyethyl starch, a clay, such as bentonite or VEEGUM HV. Generally, the bases of a suspension type lotion can be used alone or in mixture of two or more thereof.

Examples of bases of an emulsion type lotion include bases in which water and an oily substance, for example, a fatty acid, such as stearic acid, behenic acid, or oleic acid, or a higher alcohol, such as stearyl alcohol, cetanol, or behenyl alcohol, are emulsified. Generally, the bases of an emulsion type lotion can be used alone or in mixture of two or more thereof.

Examples of bases of a solution type lotion include water, and alcohols, such as ethanol, glycerol, and propylene glycol. Generally, the bases of a solution type lotion can be used alone or in mixture of two or more thereof.

A lotion can be produced, for example, by adding various base components to purified water, mixing and stirring the mixture, and then adding an active ingredient and additives thereto and mixing the mixture, followed by filtration, as required.

When the dosage form is liniment, examples of bases thereof include vegetable oils, such as olive oil, sesame oil, almond oil, cottonseed oil, and turpentine oil, alcohols, such as ethanol, propanol, and isopropanol, and mixtures thereof with water. Generally, the bases of a liniment can be used alone or in mixture of two or more thereof.

A liniment can be produced by dissolving an active ingredient in a base, and further adding desired components thereto and mixing the mixture.

When the dosage form is cataplasm, example of bases thereof include water soluble polymer compounds, such as polyacrylic acid, salts thereof, polyvinyl alcohol, and polyvinyl pyrrolidone, and crosslinked bodies, for example, a base in which such a water soluble polymer compound is crosslinked by a polyvalent metal salt, such as alum, and a base in which such a water soluble polymer compound is crosslinked by a physical treatment, such as radiation exposure . Generally, the bases of a cataplasm can be used alone or in mixture of two or more thereof.

The cataplasm can be produced by mixing an active ingredient, a base, and desired additives, and heating the mixture, followed by cooling.

For the plaster, the patch, and the emplastrum, examples of ingredients include support, such as a nonwoven fabric; an elastic body, such as a natural gum, styrene-butadiene gum (SBR), butyl gum, polyisobutylene, polyvinyl alkyl ether, polyurethane, dimethylpolysiloxane, styrene-isoprene-styrene gum, or isoprene gum; a filler, such as zinc flower, titanium oxide, or silica; a tackifier that is compatible with elastic bodies, such as a terpene resin, a rosin or an esters thereof, or a phenol resin; a release treatment agent, such as vinyl acetate, a silicone resin, or polyvinyl chloride; a softening agent, such as liquid paraffin or a process oil; and an aging retardant, such as dibutyl hydroxy toluene (BHT), are exemplified. These components can be used alone or in mixture of two or more thereof.

The plaster, patch, emplastrum, and the like can be produced by an ordinary method, such as a solution method and a hot press method. Specifically, for example, in the case of the hot press method, an active ingredient and various components are uniformly kneaded using a roll machine or the like, and the mixture is applied on a release paper into a uniform thickness using a calender with heat and pressure applied to form a medical agent-containing layer, which is then laminated and closely attached on a surface of a support, thereby the plaster, patch, emplastrum, and the like can be produced.

Also in the case of a gel, liquid, tape, and the like, the base thereof may usually be any one that is used in an external preparation, and is not particularly limited.

### First Medical Device

The first medical device according to the present invention is a medical device for preventing or treating allergic conjunctivitis.

The first medical device according to the present invention includes an breathable mask portion covering the nasal cavity of a subject in need of prevention or treatment of allergic conjunctivitis, ear loop parts that are provided on the breathable mask portion, and a composite particle that is detachably attached to the breathable mask portion, the composite particle containing one or more titanium oxide particles, one or more metal particles, and one or more calcium phosphate particles.

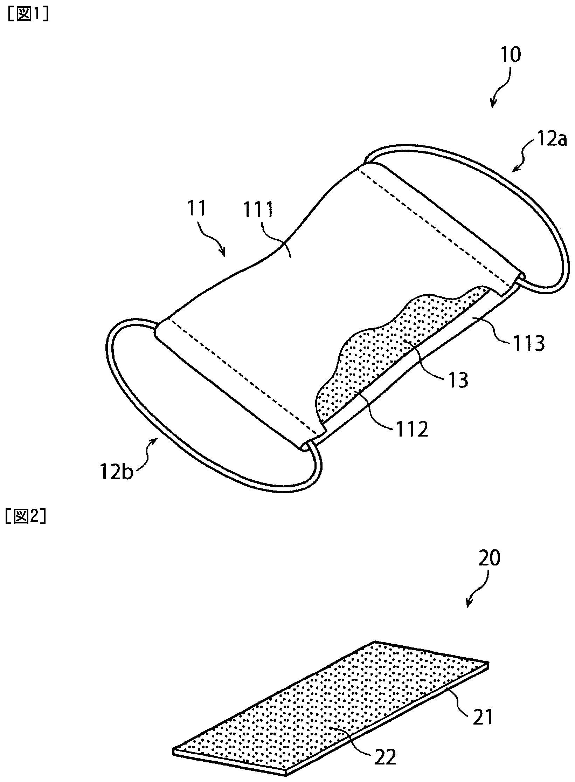

An embodiment of the first medical device according to the present invention will be described below with reference to a drawing. Fig. 1 is a diagram illustrating an embodiment of the first medical agent device according to the present invention with a part cut away.

As shown in Fig. 1, a medical device 10 according to this embodiment includes an breathable mask portion 11 covering the nasal cavity of a subject in need of prevention or treatment of allergic conjunctivitis, ear loop portions 12a and 12b that are respectively provided on both the ends of the breathable mask portion 11, and composite particles 13 that are releasably attached to the breathable mask portion 11.

When the medical device 10 is attached to a subject, the ear loop portion 12a is hung on one ear of the subject and the ear loop portion 12b is hung on the other ear of the subject, thereby at least the nasal cavity of the face of the subject is covered by the breathable mask portion 11. When the medical device 10 is attached to the subject, the mouth, in addition to the nasal cavity, of the subject may be covered by the breathable mask portion 11.

As shown in Fig. 1, the form of the medical device 10 is a flat mask form, but the form of the medical device 10 is not limited to such a flat mask form, and may be a pleated mask form, a three-dimensional mask form, and other mask forms.

The breathable mask portion 11 is a mask body covering the nasal cavity of a subject in need of prevention or treatment of allergic conjunctivitis, and has air permeability. The air permeability of the breathable mask portion 11 can be appropriately adjusted to the extent that the subject who wears the medical device 10 can breathe. The air permeability of the breathable mask portion 11 is, for example, 5 to 150 cm³/cm²·sec, and preferably 30 to 100 cm³/cm²·sec. The air permeability is measured, for example, according to JIS L10968.27.1A (Frazier type method).

The breathable mask portion 11 is formed of a plurality of breathable sheet members that are superimposed. The edges of the plurality of breathable sheet members are bonded by a known bonding method, such as heat sealing, ultrasonic welding, or adhesive. The breathable mask portion 11 includes a first breathable sheet member 111, a second breathable sheet member 112, and a third breathable sheet member 113 that are superimposed in this order. When the medical device 10 is attached to a subject, the first breathable sheet member 111 is placed on the side of the subject face, and the third breathable sheet member 113 is placed on the side of external air. The number of the breathable sheet members constituting the breathable mask portion 11 can be appropriately varied. For example, one or two or more breathable sheet members may be provided between the first breathable sheet member 111 and the second breathable sheet member 112. In addition, one or two or more breathable sheet members may be provided between the second breathable sheet member 112 and the third breathable sheet member 113.

Each breathable sheet member can be formed of, for example, a nonwoven fabric, a woven fabric, or a knitted fabric. Examples of fibers constituting each breathable sheet member include synthetic fibers, regenerated fibers, and natural fibers. Examples of synthetic fibers include polyolefin fibers, such as polyethylene and polypropylene, polyester fibers, such as polyethylene terephthalate and polybutylene terephthalate, and a polyamide fiber, such as nylon. The synthetic fiber may be a composite fiber, such as a sheath-core-type fiber. Examples of regenerated fibers include rayon and acetate. An example of a natural fiber is cotton. Examples of nonwoven fabrics include a spunbonded nonwoven fabric, a thermobonded nonwoven fabric, a spunlaced nonwoven fabric, an air-through nonwoven fabric, a meltblown nonwoven fabric, and a needle-punched nonwoven fabric. An example of a woven fabric is gauze. The nonwoven fabric may have a multilayer structure having two or more layers. Examples of such multilayer structures include an SS structure (spunbonded-spunbonded two-layer structure) and an SMS structure (spunbonded-meltblown-spunbonded three-layer structure).

A basis weight of each breathable sheet member can be adjusted in the same degree as in breathable sheet members used in commercially available house use or medical use masks. Basis weights of the first breathable sheet member 111 and the third breathable sheet member 113 can be adjusted from the viewpoint of, for example, the air permeability. A basis weight of the second breathable sheet member 112 can be adjusted from the viewpoint of, for example, the filtering property. When one or two or more breathable sheet members are provided between the first breathable sheet member 111 and the second breathable sheet member 112, or one or two or more breathable sheet members are provided between the second breathable sheet member 112 and the third breathable sheet member 113, the basis weights of these breathable sheet members can be adjusted from the viewpoint of, for example, the air permeability or the filtering property.

The ear loop portions 12a and 12b are each formed of, for example, a string-shaped member. The string-shaped member preferably has elasticity. The string-shaped member is, for example, a rubber or plastic string-shaped member having elasticity. Both the ends of the ear loop portions 12a and 12b are fixedly attached to the breathable mask portion 11, for example, by a bonding method, such as sewing, thereby forming a loop to be hung on the ears of a subject.

The composite particle 13 contains one or more titanium oxide particles, one or more metal particles, and one or more calcium phosphate particles. The foregoing description applies to the composite particle.

The composite particles 13 are attached to the breathable mask portion 11 so as to be released by the breath of a subject who wears the medical device 10. Accordingly, when a subject who wears the medical device 10 breathes, a part of multiple composite particles 13 attached to the breathable mask portion 11 is released and administered to the intranasal mucosa of the subject. That is, the composite particles 13 can be administered to the intranasal mucosa of a subject who wears the medical device 10 by using the breath of the subject, and thus allergic conjunctivitis of the subject can be prevented or treated.

The subject to which the medical device 10 is applied is not particularly limited as long as the subject needs to prevent or treat allergic conjunctivitis, and the subject is generally an allergic conjunctivitis patient.

The allergic conjunctivitis to which the medical device 10 is applied is not particularly limited as long as it causes inflammation in the conjunctiva and is accompanied by a symptom, such as eye itching, hyperemia, foreign body sensation, eye discharge, or easy-to-appear tears. The allergic conjunctivitis to which the medical device 10 is applied includes perennial allergic conjunctivitis and seasonal allergic conjunctivitis. The medical device 10 can exert an excellent prevention or treatment effect on allergic conjunctivitis that has been considered difficult to prevent or treat, in particular, on perennial allergic conjunctivitis which is initiated by house dust, mites, mold, or the like or on seasonal allergic conjunctivitis which is initiated by pollen of a plant, such as Japanese cedar, Japanese cypress, ragweed, Dactylis glomerata, Alnus, or wormwood.

In this embodiment, the composite particles 13 are detachably attached to the second breathable sheet member 112 among the plurality of sheet members constituting the breathable mask portion 11. The sheet member on which the composite particles 13 are releasably attached is not limited to the second breathable sheet member 112, and may be another breathable sheet member. In addition, the composite particles 13 can be detachably attached to two or more breathable sheet members.

The total attached amount of the composite particles per unit area of the breathable sheet member is not particularly limited, but the lower limit of the total attached amount is generally 1 g/m², preferably 2 g/m², further preferably 3 g/m², furthermore preferably 4 g/m², and furthermore preferably 5 g/m², and the upper limit of the total attached amount is generally 20 g/m², preferably 15 g/m², and further preferably 10 g/m².

The composite particles 13 can be detachably attached to a breathable sheet member, for example, via a binder resin. For example, a mixture liquid containing the composite particles 13 and a binder resin is supplied to an breathable sheet member, or an breathable sheet member is immersed in a mixture liquid containing the composite particles 13 and a binder resin, and then the breathable sheet member is dried, thereby an breathable sheet member to which the composite particles 13 are releasably attached via the binder resin can be produced. In addition, a mixture liquid containing the composite particles 13 and a binder resin is supplied to an original sheet of an air permeability sheet, or an original sheet of an air permeability sheet is immersed in a mixture liquid containing the composite particles 13 and a binder resin, then the original sheet of the air permeability sheet is dried, and then an breathable sheet member is cut from the original sheet of the air permeability sheet, thereby an breathable sheet member to which the composite particles 13 are releasably attached via the binder resin can be produced.

The amount of the binder resin contained in the mixture liquid is preferably 20 to 90 parts by mass, further preferably 30 to 85 parts by mass, and furthermore preferably 40 to 80 parts by mass relative to 100 parts by mass of the composite particles. The amount of the binder resin contained in the breathable sheet member is the same.

The total attached amount of the composite particles and the binder resin per unit area of the breathable sheet member is not particularly limited, but the lower limit of the total attached amount is generally 2 g/m², preferably 3 g/m², further preferably 4 g/m², furthermore preferably 5 g/m², and furthermore preferably 6 g/m², and the upper limit of the total attached amount is generally 30 g/m², preferably 25 g/m², further preferably 20 g/m², and furthermore preferably 15 g/m².

As a binder resin, one of known resins having adhesiveness can be used alone or two or more thereof can be used in combination. Examples of binder resins include: natural adhesive pastes or natural resins, such as gelatin, gum arabic, shellac, dammar, elemi, and sandarac; semisynthetic adhesive pastes or semisynthetic resins, such as methylcellulose, ethylcellulose, nitrocellulose, carboxymethylcellulose, and acetates; synthetic adhesive pastes or synthetic resins, for example, polyester resins, such as isophthalic-, terephthalic-, bisphenolic-, and vinylester-based polyester resins, acrylic copolymer resins, such as ethylene-acrylic acid, ethylene-acrylic acid ester, acryl ester-vinyl, and methacrylic acid ester-vinyl, urethane resins formed by a reaction of an isocyanate derivative or isocyanurate derivative, such as tolylene diisocyanate, 4,4'-diphenylmethane diisocyanate, or lysin ester triisocyanate, and an isocyanate derivative or isocyanurate derivative, such as tolylene diisocyanate, with a polyol, such as polyester polyol, polyether polyol, acrylloylol, or phenolic polyol, halogenated polymers, such as polyvinyl chloride and polyvinylidene chloride, acetal resins, such as polyvinyl acetate, an ethylene-vinyl acetate copolymer, a vinyl chloride-vinyl acetate copolymer, polyacryl ester, polystyrene, and polyvinyl acetal, polycarbonate resins, a cellulose resin, such as cellulose acetate, polyolefine resins, and amino resins, such as urea resins, melamine resins, and benzoguanamine resins; silicone resins including silicone resins, such as polyalkylsiloxane, polyalkyl hydrogen siloxane, polyalkylalkenylsiloxane, polyalkylsiliconate, polyalkali alkylsiliconate, and polyalkylphenylsiloxane, as well as modified bodies, such as epoxy-modified, amino-modified, urethane-modified, alkyd-modified, and acyl-modified silicone resins, copolymers, and the like; and fluorinated resins, for example, polymers, such as tetrafluoroethylene and fluorinated vinylidene, and copolymers of such a monomer and another monomer. Among them, from the viewpoint of adhesiveness, urethane resins and silicone resins are preferred, and urethane resins are particularly preferred.

In place of the binder resin or in combination with the binder resin, one inorganic binder may be used alone or two or more inorganic binders may be used in combination. Examples of inorganic binders include products obtained by degrading a hydrolyzable silicon compound, such as an alkyl silicate, a halogenated silicon, or a partially-hydrolyzed product thereof, organic polysiloxane compounds and polycondensation products thereof, silica, colloidal silica, water glass, a silicon compound, a phosphate salt, such as zinc phosphate, metal oxides, such as zinc oxide and zirconium oxide, a biphosphate salt, cement, plaster, lime, and frit for enamel.

The wearing time of the medical device 10 is a time effective for prevention or treatment of allergic conjunctivitis. The wearing time effective for prevention or treatment of allergic conjunctivitis can be appropriately adjusted according to the amount of the composite particles releasably attached to the breathable mask portion, the degree of the allergic conjunctivitis, the dosage regimen, and the like. The wearing time of the medical device 10 per wearing is generally 30 to 120 minutes, preferably 30 to 90 minutes, and further preferably 30 to 60 minutes . The number of wearings of the medical device 10 per day is not particularly limited, but is generally once to 10 times, preferably twice to 8 times, further preferably 3 to 6 times. The interval between the wearings of the medical device 10 can be appropriated adjusted in view of, for example, the duration of the effect of prevention or treatment of allergic conjunctivitis. The duration of the effect of prevention or treatment of allergic conjunctivitis that is expected when the medical device 10 is put to the face of a subject for one day in the above wearing time per wearing at the above number of wearings per day, although may vary among individual subjects, is generally several hours to several days. The duration of the effect of prevention or treatment of allergic conjunctivitis that is expected when the medical device 10 is put to the face of a subject for one to two weeks in the above wearing time per wearing at the above number of wearings per day, although may vary among individual subjects, is generally one week to two months.

### Second Medical Device

The second medical device according to the present invention is a medical device for preventing or treating allergic conjunctivitis which is used by being inserted into the nasal cavity of a subject in need of prevention or treatment of allergic conjunctivitis.

The second medical agent device according to the present invention includes a sheet portion to be inserted into the nasal cavity of a subject and a composite particle attached to the sheet portion, the composite particle containing one or more titanium oxide particles, one or more metal particles, and one or more calcium phosphate particles.

An embodiment of the second medical device according to the present invention will be described below with reference to a drawing. Fig. 2 is a perspective view showing an embodiment of the second medical device according to the present invention.

As shown in Fig. 2, the medical device 20 according to this embodiment includes a sheet portion 21 to be inserted into the nasal cavity of a subject and composite particles 22 attached to the sheet portion 21.

The medical device 20 is used by being inserted into the nasal cavity of a subject in need of prevention or treatment of allergic conjunctivitis. When the medical device 20 is inserted into the nasal cavity of a subject, the medical device 20 may be transformed to facilitate the insertion. For example, the medical device 20 can be twisted and transformed into a shape of twisted paper string and then be inserted into the nasal cavity of a subject. The entire medical device 20 may be inserted into the nasal cavity of a subject or a part of the medical device 20 may be inserted into the nasal cavity of a subject as long as a part of the sheet portion 21 to which the composite particles 22 are attached is inserted into the nasal cavity of the subject. From the viewpoint of easiness of removal from the nasal cavity, it is preferred that a part of the medical device 20 be inserted into the nasal cavity of a subject and the remaining part be held outside the nasal cavity of the subject. The medical device 20 is preferably inserted to a rear portion (with a distance from the subnasal point of, for example, 1 to 10 cm, preferably 1 to 8 cm) in the nasal cavity. In addition, the medical device 20 is preferably inserted into the nasal cavity of a subject so that a part of the sheet portion 21 to which the composite particles 22 are attached is brought into contact with the intranasal mucosa of the subject.

The sheet portion 21 has a size that enables the insertion into the nasal cavity of a subject. The length of the sheet portion 21 is generally 50 to 300 mm, preferably 100 to 200 mm, and the width of the sheet portion 21 is generally 5 to 40 mm, preferably 10 to 20 mm. When the medical device 20 is inserted into the nasal cavity of a subject, for example, a part of the sheet portion 21 (for example, a part having a length of 1 to 5 cm) is not inserted into the nasal cavity of the subject but is held outside the nasal cavity, and the remaining part of the sheet portion 21 is inserted into the nasal cavity of the subject. The sheet portion 21 is, for example, a strip shape. The sheet portion 21 may not have air permeability, but preferably has air permeability. The air permeability of the sheet portion 21 can be appropriately adjusted to the extent that a subject with the medical device 20 inserted can breathe. The air permeability of the sheet portion 21 is, for example, 5 to 150 cm³/cm²·sec, and preferably 30 to 100 cm³/cm²·sec. The air permeability is measured according to, for example, JIS L10968.27.1A (Frazier type method).

The sheet portion 21 can be formed of, for example, a nonwoven fabric, a woven fabric, a knitted fabric, or a plastic film having air holes. Examples of fibers constituting the sheet portion 21 include synthetic fibers, regenerated fibers, and natural fibers. Examples of synthetic fibers include polyolefin fibers, such as polyethylene and polypropylene, polyester fibers, such as polyethylene terephthalate and polybutylene terephthalate, and a polyamide fiber, such as nylon. The synthetic fiber may be a composite fiber, such as a sheath-core-type fiber. Examples of regenerated fibers include rayon and acetate. An example of a natural fiber includes cotton. Examples of nonwoven fabrics include a spunbonded nonwoven fabric, a thermobonded nonwoven fabric, a spunlaced nonwoven fabric, an air-through nonwoven fabric, a meltblown nonwoven fabric, and a needle-punched nonwoven fabric. An example of a woven fabric is gauze. The nonwoven fabric may have a multilayer structure having two or more layers . Examples of such multilayer structures include an SS structure (spunbonded-spunbonded two-layer structure) and an SMS structure (spunbonded-meltblown-spunbonded three-layer structure).

The composite particle 22 contains one or more titanium oxide particles, one or more metal particles, and one or more calcium phosphate particles. The foregoing description applies to the composite particle.

The composite particles 22 may be attached to the sheet portion 21 so as to be releasable by the breath of a subject having the medical device 20 inserted in the nasal cavity, or may be attached to the sheet portion 21 so as not to be released by the breath of a subject having the medical device 20 inserted in the nasal cavity.

When the medical device 20 is inserted into the nasal cavity of a subject, the composite particles 22 attached to the sheet portion 21 are brought into contact with or are attached to the intranasal mucosa of the subject. Accordingly, the composite particles 22 can be administered to the intranasal mucosa of the subject even without the use of the breath of the subject, thereby allergic conjunctivitis of the subject can be prevented or treated.

The subject to whom the medical device 20 is applied is not particularly limited as long as the subject needs to prevent or treat allergic conjunctivitis, but the subject is generally an allergic conjunctivitis patient, and is preferably an allergic conjunctivitis patient.

The allergic conjunctivitis to which the medical device 20 is applied is not particularly limited as long as it causes inflammation in the conjunctiva and is accompanied by a symptom, such as eye itching, hyperemia, foreign body sensation, eye discharge, or easy-to-appear tears. The allergic conjunctivitis to which the medical device 20 is applied may be perennial allergic conjunctivitis or may be seasonal allergic conjunctivitis. The medical device 20 can exert an excellent prevention or treatment effect on allergic conjunctivitis that has been considered difficult to prevent or treat, in particular, on perennial allergic conjunctivitis which is initiated by house dust, mites, mold, or the like or on seasonal allergic conjunctivitis which is initiated by pollen of a plant, such as Japanese cedar, Japanese cypress, ragweed, Dactylis glomerata, Alnus, or wormwood.

In the case where the composite particles 22 are attached to the sheet portion 21 so as to be releasable by the breath of a subject having the medical device 20 inserted in the nasal cavity, as the subject having the medical device 20 inserted in the nasal cavity breathes, a part of a large number of the composite particles 22 attached to the sheet portion 21 is released to be attached to the intranasal mucosa of the subject. Accordingly, the composite particles 22 can be administered to the intranasal mucosa of the subject by using the breath of subject having the medical device 20 inserted in the nasal cavity, thereby allergic conjunctivitis of the subject can be prevented or treated.

The total attached amount of the composite particles per unit area of the sheet portion 21 is not particularly limited, but the lower limit of the total attached amount is generally 1 g/m², preferably 2 g/m², further preferably 3 g/m², furthermore preferably 4 g/m², and furthermore preferably 5 g/m², and the upper limit of the total attached amount is generally 20 g/m², preferably 15 g/m², and further preferably 10 g/m².

The composite particles 22 can be attached to the sheet portion 21, for example, via a binder resin. For example, a mixture liquid containing the composite particles 22 and a binder resin is supplied to a sheet member, or a sheet member is immersed in a mixture liquid containing the composite particles 22 and a binder resin, and then the sheet member is dried, thereby an sheet member 21 to which the composite particles 22 are attached via the binder resin can be produced. In addition, a mixture liquid containing the composite particles 22 and a binder resin is supplied to an original sheet, or an original sheet is immersed in a mixture liquid containing the composite particles 22 and a binder resin, then the original sheet is dried, and then a sheet member is cut from the original sheet, thereby a sheet member 21 to which the composite particles 22 are attached via the binder resin can be produced.

The amount of the binder resin contained in the mixture liquid is preferably 20 to 90 parts by mass, further preferably 30 to 85 parts by mass, and furthermore preferably 40 to 80 parts by mass relative to 100 parts by mass of the composite particles. The amount of the binder resin contained in the sheet portion 21 is the same.

The total attached amount of the composite particles and the binder resin per unit area of the sheet portion 21 is not particularly limited, but the lower limit of the total attached amount is generally 2 g/m², preferably 3 g/m², further preferably 4 g/m², furthermore preferably 5 g/m², and furthermore preferably 6 g/m², and the upper limit of the total attached amount generally 30 g/m², preferably 25 g/m², further preferably 20 g/m², and furthermore preferably 15 g/m².

As the binder resin, one known resin having adhesiveness can be used alone or two or more such resins can be used in combination. Specific examples of the binder resins are the same with the specific examples described for the first medical device.

In place of the binder resin or in addition to the binder resin, one inorganic binder may be used alone or two or more inorganic binders may be used in combination. Specific examples of the inorganic binders are the same with the specific examples described for the first medical device.

The insertion time of the medical device 20 in the nasal cavity is a time effective for prevention or treatment of allergic conjunctivitis. The insertion time effective for prevention or treatment of allergic conjunctivitis can be appropriately adjusted according to the amount of the composite particles 22 attached to the sheet portion 21, the degree of the allergic conjunctivitis, the dosage regimen, and the like. The insertion time of the medical device 20 in the nasal cavity per insertion is generally 10 to 60 minutes, preferably 20 to 60 minutes, and further preferably 30 to 45 minutes. The number of insertions of the medical device 20 in the nasal cavity per day is not particularly limited, but is generally once to 3 times, preferably once to twice, and further preferably once. The interval between the insertions of the medical device 20 in the nasal cavity can be appropriately adjusted in view of, for example, the duration of the effect of prevention or treatment of allergic conjunctivitis.
The duration of the effect of prevention or treatment of allergic conjunctivitis that is expected when the medical device 20 is inserted in the nasal cavity of a subject for one day in the above insertion time per insertion at the above number of insertions per day, although varies among individual subject, is generally several hours to several days. The duration of the effect of prevention or treatment of allergic conjunctivitis that is expected when the medical device 20 is inserted to the nasal cavity of a subject for one to two weeks in the above insertion time per insertion at the above number of insertions per day, although may vary among individual subjects, is generally one week to two months.

### Method

The method according to the present invention is a method for preventing or treating allergic conjunctivitis.

The method according to the present invention includes a step of administering a composite particle to the intranasal mucosa of a subject in need of prevention or treatment of allergic conjunctivitis, the composite particle containing one or more titanium oxide particles, one or more metal particles, and one or more calcium phosphate particles. The foregoing description applies to the composite particle.

The subject to whom the method according to the present invention is applied is not particularly limited as long as the subject needs to prevent or treat allergic conjunctivitis, but is generally an allergic conjunctivitis patient.

The allergic conjunctivitis to which the method according to the present invention is applied is not particularly limited as long as it causes inflammation in the conjunctiva and is accompanied by a symptom, such as eye itching, hyperemia, foreign body sensation, eye discharge, or easy-to-appear tears. The allergic conjunctivitis to which the method according to the present invention is applied may be perennial allergic conjunctivitis or may be seasonal allergic conjunctivitis. The method according to the present invention can exert an excellent prevention or treatment effect on allergic conjunctivitis that has been considered difficult to prevent or treat, in particular, on perennial allergic conjunctivitis which is initiated by house dust, mites, mold, or the like or on seasonal allergic conjunctivitis which is initiated by pollen of a plant, such as Japanese cedar, Japanese cypress, ragweed, Dactylis glomerata, Alnus, or wormwood.

The dose of the composite particles is an amount effective for prevention or treatment of allergic conjunctivitis. The amount effective for prevention or treatment of allergic conjunctivitis can be appropriately adjusted according to the dosage form of the composite particles administered, the degree of the allergic conjunctivitis, the dosage regimen, and the like. The dose of the composite particles per administration is generally 0.1 to 10 µg, preferably 0.2 to 5 µg, and further preferably 0.4 to 4 µg. The number of administrations of the composite particles per day is not particularly limited, but is generally once to 5 times, preferably once to 3 times, further preferably once to twice. The interval between administrations of the composite particles can be appropriately adjusted in view of, for example, the duration of the effect of prevention or treatment of allergic conjunctivitis. The duration of the effect of prevention or treatment of allergic conjunctivitis that is expected when the composite particles are administered to a subject for one day in the above dose per administration at the above number of administrations per day, although may vary among individual subjects, is generally several hours to several days. The duration of the effect of prevention or treatment of allergic conjunctivitis that is expected when the composite particle is administered to a subject for one to two weeks in the above dose per administration at the above number of administrations per day, although may vary among individual subjects, is generally one week to two months.

In an embodiment of the method according to the present invention, the pharmaceutical preparation according to the present invention is administered to the intranasal mucosa of a subject in an administration step, thereby the composite particles are administered to the intranasal mucosa of the subject.

In an embodiment of the method according to the present invention, the first medical device according to the present invention is put to the face of a subject in an administration step, thereby the composite particles are administered to the intranasal mucosa of the subject.

In an embodiment of the method according to the present invention, the second medical device according to the present invention is inserted into the nasal cavity of a subject in an administration step, thereby the composite particles are administered to the intranasal mucosa of the subject.

### EXAMPLES

The present invention will be described in more detail below with reference to Production Examples and Test Example. However, the scope according to the present invention is not to be limited to the Production Examples and Test Example.

### Production Example 1: Production of Composite Particles

In this Production Example, composite particles each containing one or more titanium oxide particles, one or more silver particles, and one or more hydroxyapatite particles were produced using a titanium oxide powder, a silver powder, and a hydroxyapatite powder as raw material powders.

In this Production Example, two types of composite particles M1 and M2 were produced. The composite particles M1 and M2 are different in terms of the ratio of titanium oxide, silver, and hydroxyapatite contained. The composite particles M1 and M2 were produced in the same manner as for "earthplus" (trade name) manufactured and sold by Shinsyu Ceramics Co., Ltd. The production of the composite particles M1 and M2 was outsourced to Shinsyu Ceramics Co., Ltd.

The raw material powders shown in Table 1 were provided. The particle size of the titanium oxide powder is a value measured using a transmission electron microscope (TEM) or a scanning electron microscope (SEM), the particle size of the silver powder is a value calculated based on the specific surface area, and the particle size of the hydroxyapatite powder is a value measured by a laser diffraction scattering method. Since the silver powder was stored in a freezer until use, the agglomeration of the silver particles contained in the silver powder was suppressed.

**[Table 1]**

| Raw Material Powder | | | |
|---|---|---|---|
| | Anatase Type Titanium Oxide powder | Hydroxyapatite Powder | Silver Powder |
| CAS No. | 13463-67-7 | 10103-46-5 | 7440-22-4 |
| Punity | 95 wt% or more | 95 wt% or more | 95 wt% or more |
| Shape | powder | powder | powder |
| Color | white | White | - |
| Smell | none | none | None |
| Spcific gravity | 3.9 | 2.7 | 10.5 |
| Particle size of primary particles | 0.03 to 0.1 µm | 0.1 to 0.2 µm | 1.1 to 1.9 µm |
| Particle size of secondary particle | 1 to 2 µm | 4 to 5 µm | - |
| pH | 6 to 8 | - | - |
| Melting point | 1823°C, or more | 1670°C, or more | 962°C, or more |

The titanium oxide powder, the silver powder, the hydroxyapatite powder, and a polycarboxylic acid-based dispersant were mixed in water using a commercially available wet bead mill ("Star Mill LME" manufactured by Ashizawa Finetech Ltd.) to composite one or more titanium oxide particles contained in the titanium oxide powder, one or more silver particles contained in the silver powder, and one or more hydroxyapatite particles contained in the hydroxyapatite powder, thereby producing a suspension (slurry) of composite particles. The wet bead mill used can finely pulverize titanium oxide particles, silver particles, and hydroxyapatite particles contained in raw material powders into nanoparticle or submicron particles while dispersing the particles, and can simultaneously composite the finely pulverized particles.

The conditions in compositing the particles using the wet bead mill are as follows.
Total amount of raw material powders added: 4 kg or more Volume of cylinder: 3.3 L
Beads: zirconia beads (diameter: 0.5 mm, mass: 0.37 mg) Flow rate of liquid: 2 L/minute
Circumferential speed of blade in cylinder: 540 m/minute Liquid temperature: 35 to 45°C
Mixing time per Kg raw material powders: 30 to 40 minutes (about 36 minutes)

The total amount of the titanium oxide powder, the silver powder, and the hydroxyapatite powder blended was adjusted to 35 parts by mass relative to 65 parts by mass of water. The amount of polycarboxylic acid-based dispersant blended was adjusted to 0.5 parts by mass relative to 35 parts by mass of the total amount of the titanium oxide powder, the silver powder, and the hydroxyapatite powder blended.

In the production of the composite particles M1, the amount of the titanium oxide powder blended was adjusted to about 160 parts by mass (155 to 165 parts by mass) relative to 1 part by mass of the silver powder, and the amount of the hydroxyapatite powder blended was adjusted to about 40 parts by mass (39 to 41 parts by mass) relative to 1 part by mass of the silver powder.

In the production of the composite particles M2, the amount of the titanium oxide powder blended was adjusted to about 30 parts by mass (29 to 31 parts by mass) relative to 1 part by mass of the silver powder, and the amount of the hydroxyapatite powder blended was adjusted to about 3 parts by mass (2.5 to 3.5 parts by mass) relative to 1 part by mass of the silver powder.

By drying the suspension (slurry) of the composite particles, the composite particles M1 and M2 were produced. The particle sizes of the composite particles M1 and M2 measured by dynamic light scattering were 200 to 500 nm. The median diameters (d50) of the composite particles M1 and M2 measured on the volume basis by dynamic light scattering were about 300 nm. The particle size by dynamic light scattering was measured by using a commercially available dynamic light scattering particle size distribution analyzer, specifically, a dynamic light scattering Nanotrac particle size distribution analyzer, "UPA-EX150" (manufactured by Nikkiso Co. Ltd.).

### Production Example 2: Production of Composite Particle-attached Nonwoven Fabric

To the suspension (slurry) of the composite particles M1 obtained in Production Example 1, a binder resin was added to prepare a mixture liquid, and then a polyester spunbonded nonwoven fabric was immersed in the mixture liquid to impregnate the nonwoven fabric with the mixture liquid. After immersion, the nonwoven fabric was taken out of the mixture liquid, and was pressed with a roller to squeeze out the excessive mixture liquid. After pressing, the nonwoven fabric was dried at about 130°C for about 1 minute, thereby producing a composite particle-attached nonwoven fabric N1. As the binder resin, a urethane resin (C₃H₇NO₂/NH₂COOC₂H₅) was used.

By adjusting the concentration of the composite particles M1 and the binder resin in the mixture liquid, the total attached amount of the composite particles M1 and the binder resin (the total foxed amount) per unit area of the composite particle-attached nonwoven fabric N1 was adjusted to 4 g/m², 6 g/m², 8 g/m, or 10 g/m².

The breakdown of 4 g/m² is 2.27 g/m² of titanium oxide, 0.571 g/m² of hydroxyapatite, 0.014 g/m² of silver, and 1.14 g/m² of the binder resin.

The breakdown of 6 g/m² is 3.41 g/m² of titanium oxide, 0.857 g/m² of hydroxyapatite, 0.021 g/m² of silver, and 1.71 g/m² of the binder resin.

The breakdown of 8 g/m² is 4.54 g/m² of titanium oxide, 1.143 g/m² of hydroxyapatite, 0.029 g/m² of silver, and 2.29 g/m² of the binder resin.

The breakdown of 10 g/m² is 5.68 g/m² of titanium oxide, 1.428 g/m² of hydroxyapatite, 0.036 g/m² of silver, and 2.86 g/m² of the binder resin.

A composite particle-attached nonwoven fabric N2 was produced in the same manner as the above except that a suspension of the composite particles M2 was used in place of the suspension of the composite particles M1.

By adjusting the concentration of the composite particles M2 and the binder resin in the mixture liquid, the total attached amount of the composite particles M2 and the binder resin (total fixed amount) per unit area of the composite particle-attached nonwoven fabric N2 was adjusted to 13.5 g/m².

The breakdown of 13.5 g/m² is 6.525 g/m² of titanium oxide, 0.750 g/m² of hydroxyapatite, 0.225 g/m² of silver, and 6.00 g/m² of the binder resin.

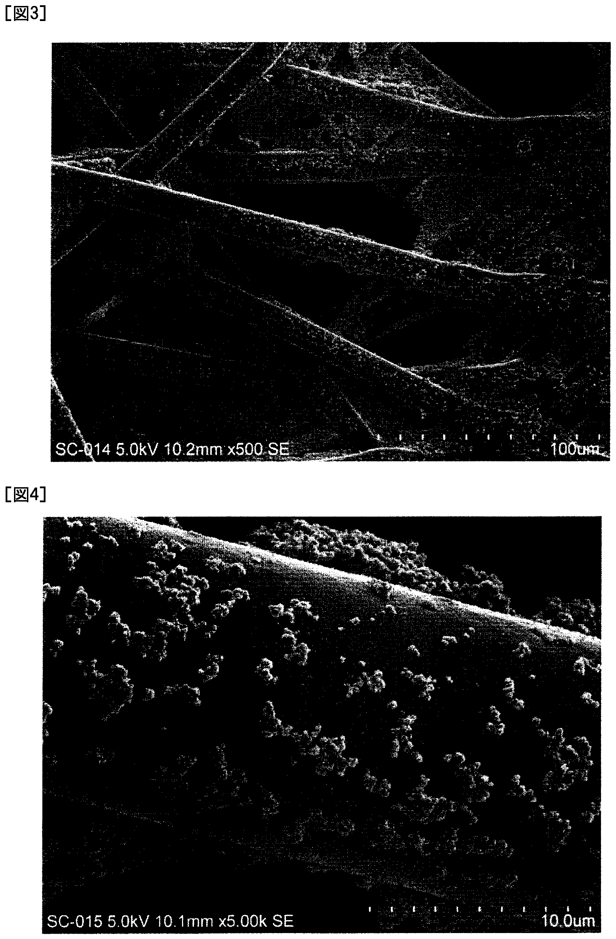

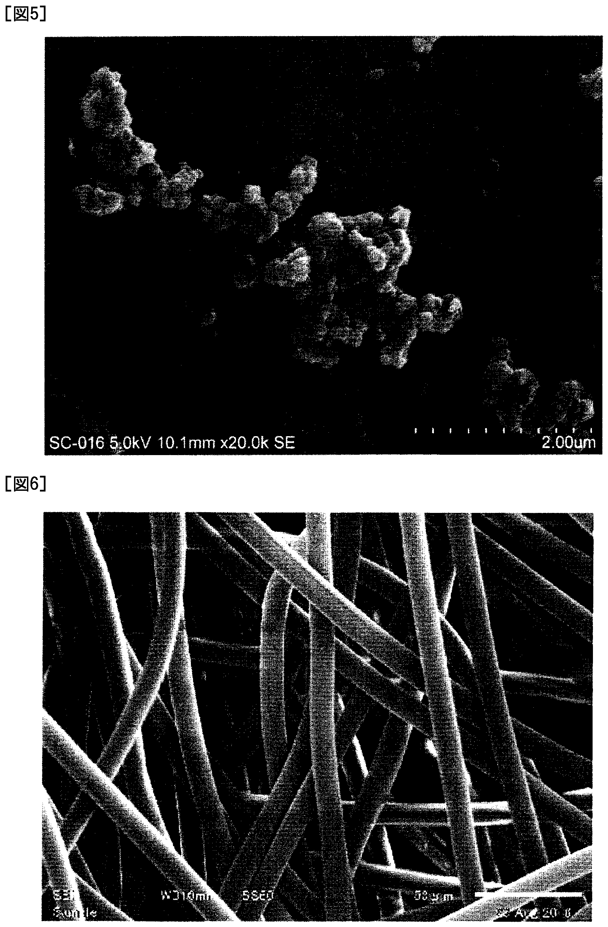

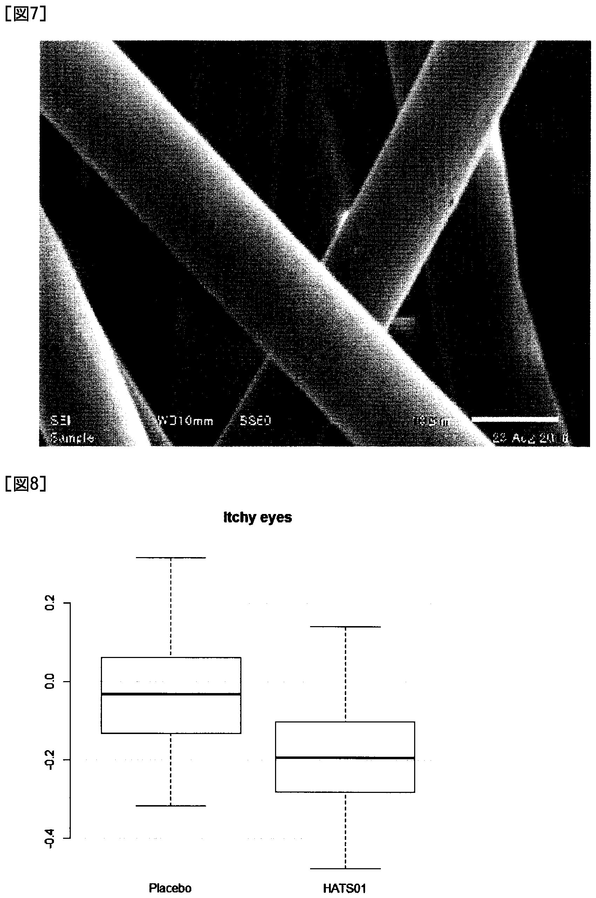

The composite particle-attached nonwoven fabric N1 in which the total attached amount of the composite particles M1 and the binder resin (total fixed amount) of 4 g/m² was observed with an electron microscope. The results of the electron microscope observation were shown in Figs. 3 to 5. Fig. 3 shows a result of observation at ×500, Fig. 4 shows a result of observation at ×5000, and Fig. 5 shows a result of observation at ×20000. As shown in Figs. 3 to 5, it was confirmed that the composite particles M1 were attached to the nonwoven fabric. Note that, as a reference, results of electron microscope observation of a nonwoven fabric with no composite particle attached are shown in Fig. 6 and Fig. 7. Fig. 6 shows a result of observation at ×500, and Fig. 7 shows a result of observation at ×1000.

### Production Example 3: Production of Composite Particle-attached Sheet

The composite particle-attached nonwoven fabric produced in Production Example 2 was cut to produce a composite particle-attached sheet of a strip shape having 1 cm width and 10 cm length. As the composite particle-attached nonwoven fabric, a composite particle-attached nonwoven fabric N1 in which the total attached amount of the composite particles M1 and the binder resin (total fixed amount) of 4 g/m² was used.

### Production Example 4: Production of Composite Particle-attached Mask

A polypropylene spunbonded nonwoven fabric, a polypropylene meltblown nonwoven fabric, a composite particle-attached nonwoven fabric, and a polypropylene spunbonded nonwoven fabric were laminated in this order from the external air side to produce a composite particle-attached mask. As the composite particle-attached nonwoven fabric, a composite particle-attached nonwoven fabric N2 in which the total attached amount of the composite particles M2 and the binder resin (total fixed amount) of 13.5 g/m² was used.

### Production Example 5: Production of Composite Particles-Cointaining Ointment

Petroleum for medical purposes (Pharmacopeial white petroleum, manufactured by KENEI Pharmaceutical, Co., Ltd.) and the composite particles M1 were mixed to produce an ointment containing 1% by weight of the composite particles M1.

### Test Example

### 1. Subject

According to Hana Arerugi Sinryou Gaidorain (nasal allergy medical care guideline) - Tsuunensei Bien to Kafunsyou (perennial rhinitis and pollinosis) - 2016 (revised 8th edition), patients complaining allergic conjunctivitis symptoms were collected from among patients who were diagnosed as seasonal allergic rhinitis (pollinosis).

### 2. Test Method

### 1) Design

Under the subjects' consent, the subjects were divided into a group of using the composite particle-attached nonwoven fabric (hereinafter may be referred to as HATS) according to the present invention and a group of using a control according to a random allocation table based on sex stratification.

HATS using group: effectiveness of investigational device, pre-observation period (2 weeks) and safety observation

Control using group: comparative observation (2 weeks)

Collection of 50 cases each, 100 cases in total, was planned, and a parallel-group comparison study was performed under a double blind test.

### 2) Test Device

Investigational device: a sheet obtained by cutting the composite particle-attached nonwoven fabric (Hydrosilver Titanium (registered trademark) sheet (HATS-01)) produced in Production Example 2 into a strip of 2 cm width, 20 cm length, and 0.4 mm thickness

Control: a sheet obtained by cutting an additive-free nonwoven fabric with no composite particle attached into a strip of 2 cm width, 20 cm length, and 0.4 mm thickness

### 3) Use Method and Use Period

The investigational device (HATS) and the control were each manually inserted into the nasal cavities (both). The investigational device (HATS) and the control were each used by insertion for 30 minutes per day during a period from rising to 10. They were instructed to expose 1 to 2 cm of the sheet outside the entrance of the nasal cavity, and to pick the sheet out when removing the sheet.

They were instructed not to roll the sheet into a small piece, not to roll it into a twisted thin string, or not to cut it into a short piece for insertion, and they were instructed not to reuse the used sheet, for example, after washing. The use period was two continuous weeks.

### 4) Combination Therapy, Combined Drug

Any combined drug that had conventionally been used was able to be used. In addition, regarding a combined drug that had conventionally been used only when any allergic symptom worsened or was severe, they were instructed to write the name of the drug and the time of taking the drug in a symptom diary.

### 5) Consent of Patients

A principal investigator explained an informed consent form approved in the institutional review board and other documents for explanation to the patients and received 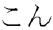 consent in writing or orally.

### 3. Observation Items

### 1) Background of Patients

The following items were examined: answer to a medical interview about age, sex, allergic history, etc., examination items, such as eye symptom and intranasal eosinophil test, body measurement, vital signs, nasal cavity finding score, allergic test, hematological test, biochemical test, urine test, allergen identification test (six items of Japanese cedar / Japanese cypress / ragweed / Dactylis glomerate /Alnus (genus) / wormwood), and urine pregnancy test.

### 2) Symptom Diary

The patients were required to write a diary every day. The patients were to write eye itching scores in three time periods of the morning (from 6 to 12), the daytime (from 12 to 18), and the evening (from 18 to 22). The eye itching score was shown in detail in Table 2. When the use of any combined drug was permitted, the patient was to write the name and the use time of the drug in the form of free writing.

**[Table 2]**

| Eve itching score | |
|---|---|
| Score | Standard |
| 0 | Less than 1 (no eye itching) |
| 1 | No eye itching but some hyperemia |
| 2 | Some eye itching and some hyperemia |
| 3 | Very strong eye itching |
| 4 | Difficult to open eyes due to eye itching |

### 3) Amount of Scattering Pollen

The amount of scattering pollen obtained by the pollen observation system of the Ministry of the Environment (http://kafun.taiki.go.jp/) was used. In the pollen observation system data of the Ministry of the Environment, the amount of scattering pollen is measured every hour, and the total amount of scattering pollen from 6 to 22 was used in this analysis.

### 4) Statistical Analysis

The HATS using group and the control using group were statistically compared in the background information. For continuous variates, the median, the 25% point, and the 75% point were indicated, and whether there was any significant difference between the two groups was checked by the Mann-Whitney test. For categorical variates, the frequency was indicated. Whether there was any significant difference between the two groups was checked by the Fisher's exact test for unordered categorical variates, and by the Cochran-Armitage test for ordered categorical variates. The evaluation was performed with a significance level of 0.00089 (0.05/56) under the Bonferroni correction method while taking into account the multiplicity of the Type I error.

The HATS using group and the control using group were compared in the variation in the eye itching score recorded in the symptom diary. A linear regression model was built while adopting, as the target variates, the variations in the number of times and the score between the morning and the evening (the number and score in the evening - the number and score in the morning) in each day in the period from the first day to the 14th day of the test. For the explanatory variates, whether the HATS using or the control using was taken as a primary endpoint, and the sex, the age, the total amount of scattering pollen on the observation day, and whether any combined drug was used were used as covariates. For building an optimum linear regression model, the model selection based on AIC was performed under the backward elimination. The regression coefficient and the 95% confidence interval of the regression coefficient were estimated as the degree of association, and the Wald test was performed for testing the presence of association. Furthermore, the goodness of fit of the built linear regression model was evaluated by the ROC analysis, and the ROC curve and the AUC value were estimated. All the statistical analyses were performed using R version 3.3.3 (R Core Team 2017, Vienna, Austria).

### 4. Results

### 1) Number of Cases to be Analyzed

The total number of cases analyzed using HATS and the control was 85 in which 41 cases belonged to the HATS using group and 44 cases to the control using group.

### 2) Comparison in Background

As a result of the investigation of the difference in background between the HATS using group and the control using group, no significant difference in background was found between the HATS using group and the control using group.

### 3) Comparative Association Study with Symptoms

An association study of the improvement in symptoms with the HATS using group and the control using group was performed, and the results of the models based on all the explanatory variates (after the model selection) were summarized in Table 3.

In addition, the distribution of the predictive value of the variation by the linear regression model after the model selection is shown in Fig. 8 as box plots. In Fig. 8, the distribution was dividedly shown for the HATS using group and the control using group (placebo group).

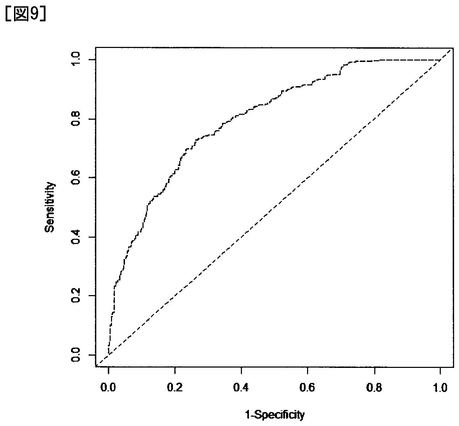

Furthermore, an ROC analysis was performed to evaluate the goodness of fit of the multivariate analysis model. The ROC curve was shown in Fig. 9.

According to the result of this analysis, the HATS using group was significantly different from the control using group in the variation in the eye itching score. In other words, it was demonstrated that the HATS using group improved the eye itching score from the morning to the evening significantly more as compared with the control using group (the regression coefficient was -0.142, the P value was 0.00268).

For the other explanatory variates, a significant worsening with increase in the amount of scattering pollen was suggested (regression coefficient = 0.015, P = 0.00395), a significant improvement trend with increase in the age was suggested (the regression coefficient = -0.006, P = 0.00473), and a significant improvement trend in the Combined drug-present group as compared with the Combined drug-absent group was suggested (the regression coefficient = -0.180, P = 0.00015).

As shown in Fig. 9, the ROC curve was drawn to be larger than AUC=0.5, and the AUC value was as large as 0.799 (0.774-0.824), and thus the goodness of discrimination between the HATS using group and the control using group was able to be demonstrated.

**[Table 3]**

| Variate | | Regression coefficient (95% confidence interval) | p-value |
|---|---|---|---|
| Constant term | - | 0.217 (0.017, 0.417) | 0.03356 |
| Medical agent administration group | HATS group | -0.142 (-0.234, -0.049) | 0.00268* |
| Amount of scattering pollen ^{a} | - | 0.015(0.005, 0.025) | 0.00395* |
| Age | - | -0.006(-0.010, -0.002) | 0.00473 |
| Presence of Combined drug use | Yes | -0.180(-0.272, -0.087) | 0.00015* |

| | | | |
|---|---|---|---|
| ^{a} Amount of scattering pollen: amount of scattering pollen per m³ | | | |

In addition, when a patient who was also diagnosed as pollinosis and complains conjunctivitis symptoms, such as eye itching, wore the composite particle-attached mask produced in Production Example 4, it was found that the eye itching was dramatically improved by wearing the mask for several ten minutes to several hours.

### Device Evaluation Example 1

Test pieces S1 to S3 having a size of 1 cm × 5 cm were produced from a polyester spunlaced nonwoven fabric ("S0040" manufactured by Yuho Co., Ltd., basis weight: 40 g/m²) in which the total attached amount of the composite particles M1 and the binder resin (total fixed amount) adjusted to 4 g/m² produced in the same manner as in Production Example 2.

The test pieces S1 to S3 were treated with heat at 160°C for 5 minutes, then was added to 30 mL of physiological saline, and was allowed to stand in a 37°C thermostatic chamber for 2 hours . The nonwoven fabric was taken out of the physiological saline, and then the physiological saline was filtered through a membrane filter (pore size: 0.1 µm).

The titanium ions and silver ions contained in the filtrate were quantified with a plasma mass spectrometry ("Agilent 7800" manufactured by Agilent Technologies, Inc.). In addition, quantification was performed in the same manner as above except that no test piece was added to 30 mL of physiological saline, and this was taken as a control. Each quantification was performed three times, and the average of the three quantified values was calculated. Note that the lower detection limit is 1.0 ppb.

The results of the quantification are shown in Tables 4 and 5.

**[Table 4]**

| Quantification Results of Titanium Ions in Filtrate (Unit: ppb) | | | | | |
|---|---|---|---|---|---|
| | | Control | Test piece | | |
| | | | S1 | S2 | S3 |
| Quantified value | 1st | <1.0 | <1.0 | <1.0 | <1.0 |
| | 2nd | <1.0 | <1.0 | <1.0 | <1.0 |
| | 3rd | <1.0 | <1.0 | <1.0 | <1.0 |
| | Average | <1.0 | <1.0 | <1.0 | <1.0 |

**[Table 5]**

| | | | | | |
|---|---|---|---|---|---|
| Quantification Results of Titanium Ions in Filtrate (Unit: ppb) | | | | | |
| | | Control | Test piece | | |
| | | | S1 | S2 | S3 |
| Quantified value | 1st | <1.0 | 3.6 | 2.1 | 4.0 |
| | 2nd | <1.0 | 3.6 | 2.0 | 4.0 |
| | 3rd | <1.0 | 3.6 | 2.0 | 3.8 |
| | Average | <1.0 | 3.6 | 2.0 | 3.9 |

The membrane filter after filtration was dissolved in a mixture liquid of hydrofluoric acid and nitric acid and treated with microwave, and the titanium element and silver element contained in the solution were quantified with a plasma emission analyzer ("PS3520UVDDII" manufactured by Hitachi High-Tech Science Corporation). In addition, quantification was performed in the same manner as above except that no test piece was added to 30 mL of physiological saline, and this was taken as a control. Each quantification was performed three times, and the average of the three quantified values was calculated. Note that the lower detection limit is 1 µg.

The results of the quantification are shown in Tables 6 and 7.

**[Table 6]**

| Quantification Result of Titanium Element (Unit: µg) | | | | | |
|---|---|---|---|---|---|
| | | Control | Test piece | | |
| | | | S1 | S2 | S3 |
| Quantified value | 1st | <1 | 1 | 1 | <1 |
| | 2nd | <1 | 1 | 1 | <1 |
| | 3rd | <1 | 1 | 1 | <1 |
| | Average | <1 | 1 | 1 | <1 |

**[Table 7]**

| Quantification Result of Silver Element (Unit: µg) | | | | | |
|---|---|---|---|---|---|
| | | Control | Test piece | | |
| | | | S1 | S2 | S3 |
| Quantified value | 1st | <1 | <1 | <1 | <1 |
| | 2nd | <1 | <1 | <1 | <1 |
| | 3rd | <1 | <1 | <1 | <1 |
| | Average | <1 | <1 | <1 | <1 |

The calculated values of the amounts of the components attached to one test piece (size: 1 cm × 5 cm) were 1.13 × 10⁻³ (g) for titanium oxide, 2.94 × 10⁻⁴ (g) for hydroxyapatite, 7.55 × 10⁻⁶ (g) for silver, and 5.71 × 10⁻⁴ (g) for the binder resin. Thus, the above results show that even if a test piece is immersed into physiological saline, titanium oxide and silver mostly remain in the nonwoven fabric. Accordingly, in a patient having a composite particle-attached sheet inserted in the nasal cavity, even if nasal mucus is generated, the composite particles mostly remain in the sheet without eluted or released, and thus, the composite particle-attached sheet is considered able to sustainably exert an effect of preventing or treating allergic conjunctivitis by being administered to the intranasal mucosa.

### Device Evaluation Example 2

A test piece having a diameter of 47 mm was produced by cutting the composite particle-attached mask produced in Production Example 4. The test piece was held so that the surface facing the external air side on wearing the mask was positioned on the upper side and the surface facing the face side on wearing the mask was positioned on the lower side, and the test piece was interposed between an upper polyethylene ring holder and a lower polyethylene ring holder (outer diameter: 47 mm, inner diameter: 20 mm). A flow meter and a suction pump were connected to the lower holder via a pipe, and preliminary suction was performed (at 5 L/minute for about 10 seconds). After the preliminary suction, a collection filter (MF-millipore VCWP09025 with a diameter of 90 mm) was connected between the lower holder and the flow meter and suction pump via a pipe, and main suction was performed (at 10 L/minute for 8 hours). The collection filter can collect particles having a particle size of 0.1 µm or more. Note that as the pipes, ones washed by an ultrasonic treatment in pure water for 3 minutes and then dried were used. The pressure (negative pressure) between the lower holder and the collection filter was measured using a digital manometer (HT-1500NH manufactured by Hodaka Corporation).

After the main suction, the holders and pipes were washed with about 40 mL of pure water, and the solid matter in the washing liquid was collected by the collection filter (MF-millipore VCWP04700 having a diameter of 47 mm).

The collection filter having a diameter of 90 mm and the collection filter having a diameter of 47 mm were each dissolved in a mixed acid solution of hydrofluoric acid and nitric acid and treated with microwave, and then titanium, silver, and hydroxyapatite contained in the solution were quantified with a plasma emission analyzer ("PS3520UVDDII" manufactured by Hitachi High-Tech Science).

A composite particle-attached mask was produced in the same manner as in Production Example 4 except that the composite particle-attached nonwoven fabric N1 in which the total attached amount of the composite particles M1 and the binder resin (total fixed amount) of 4 g/m², 6 g/m², 8 g/m² or 10 g/m² in place of the composite particle-attached nonwoven fabric N2 in which the total attached amount of the composite particles M2 and the binder resin (total fixed amount) of 13.5 g/m². Test pieces with a diameter of 47 mm were produced by cutting the produced composite particle-attached masks, and the test was performed in the same manner as above. Note that a test was also performed in which polyethylene ring holders (outer diameter: 47 mm, inner diameter: 39 mm) were used in place of the polyethylene ring holders (outer diameter: 47 mm, inner diameter: 20 mm) as the two holders placed on the upper and lower sides of the test piece. In addition, a test was also performed in which main suction was performed without preliminary suction.

The results are shown in Table 8.

**[Table 8]**

| Conditions and Results of Suction Test | | | | | | |
|---|---|---|---|---|---|---|
| Total Attached Amount (g) | Prelimi nary Suction | Inside Diameter of Holder(mm) | Negative Pressure (mmHg) | Titanium Oxide (g) | Silver (g) | Hydroxyap atic (g) |
| 13.5 | Yes | 20 | 4.5∼5.1 | 6.92×10⁻⁴ | 2.39×10⁻⁵ | 7.95×10⁻⁵ |
| 10 | No | 39 | 0.8∼0.9 | 6.82×10⁻³ | 4.32×10⁻⁵ | 1.71×10⁻³ |
| 8 | Yes | 20 | 5.4∼6.6 | 4.81×10⁻⁴ | 3.07×10⁻⁶ | 1.21×10⁻⁴ |
| 4 | Yes | 20 | 5.0∼6.4 | 2.41×10⁻⁴ | 1.48×10⁻⁶ | 6.05×10⁻⁵ |
| 4 | No | 20 | 5.2∼6.9 | 2.41×10⁻⁴ | 1.48×10⁻⁶ | 6.05×10⁻⁵ |
| 4 | No | 39 | 0.5 | 2.72×10⁻³ | 1.68×10⁻⁵ | 6.93×10⁻⁴ |

As shown in Table 8, the composite particles were released from the composite particle-attached nonwoven fabric by suction. Accordingly, it is considered that, when a subject wearing a composite particle-attached mask breathes, a part of many composite particles attached to the mask is released and is taken into the nasal cavity of the subject. In other words, it is considered that the composite particles can be administered to the intranasal mucosa of a subject wearing a composite particle-attached mask by using the breath of the subject, thereby allergic conjunctivitis of the subject can be prevented or treated.

### INDUSTRIAL APPLICABILITY

According to the present invention, a pharmaceutical preparation for preventing or treating allergic conjunctivitis which is to be administered to the intranasal mucosa, a medical device for preventing or treating allergic conjunctivitis, a method for preventing or treating allergic conjunctivitis, a composite particle for use in a method for preventing or treating allergic conjunctivitis, and use of a composite particle for producing a pharmaceutical preparation for preventing or treating allergic conjunctivitis are provided.

### REFERENCE SIGNS LIST

10: medical device, 11: breathable mask portion, 12a: ear loop portion, 12b: ear loop portion, 13: composite particle, 111: first breathable sheet member, 112: second breathable sheet member, 113: third breathable sheet member, 20: medical device, 21: sheet portion, 22: composite particle

## Claims

1. A pharmaceutical preparation for preventing or treating allergic conjunctivitis which is to be administered to an intranasal mucosa, the pharmaceutical preparation including a composite particle containing one or more titanium oxide particles, one or more metal particles, and one or more calcium phosphate particles.

2. The pharmaceutical preparation according to claim 1, wherein in the composite particle, the one or more titanium oxide particles, the one or more metal particles, and the one or more calcium phosphate particles are arranged in a three-dimensionally and randomly.

3. The pharmaceutical preparation according to claim 1 or 2, wherein at least one metal particle of the one or more metal particles is fixedly attached to at least one titanium oxide particle of the one or more titanium oxide particles.

4. The pharmaceutical preparation according to any one of claims 1 to 3, wherein the one or more metal particles are selected from the group consisting of a silver particle, a gold particle, a platinum particle, and a copper particle.

5. The pharmaceutical preparation according to any one of claims 1 to 4, wherein the one or more metal particles comprise a silver particle and the one or more calcium phosphate particles comprise a hydroxyapatite particle.

6. A medical device for preventing or treating allergic conjunctivitis, the medical device comprising
a breathable mask portion covering the nasal cavity of a subject in need of prevention or treatment of allergic conjunctivitis, an ear loop portion provided on the breathable mask portion, and a composite particle detachably attached to the breathable mask portion,
wherein the composite particle containing one or more titanium oxide particles, one or more metal particles, and one or more calcium phosphate particles.

7. The medical device according to claim 6, wherein, in the composite particle, the one or more titanium oxide particles, the one or more metal particles, and the one or more calcium phosphate particles are arranged in a three-dimensionally and randomly.

8. The medical device according to claim 6 or 7, wherein at least one metal particle of the one or more metal particles is fixedly attached to at least one titanium oxide particle of the one or more titanium oxide particles.

9. The medical device according to any one of claims 6 to 8, wherein the one or more metal particles are selected from the group consisting of a silver particle, a gold particle, a platinum particle, and a copper particle.

10. The medical device according to any one of claims 6 to 9, wherein the one or more metal particles comprise a silver particle and the one or more calcium phosphate particles comprise a hydroxyapatite particle.

11. A medical device for preventing or treating allergic conjunctivitis, which is inserted for use into the nasal cavity of a subject in need of prevention or treatment of allergic conjunctivitis, the medical device comprising
a sheet portion to be inserted into the nasal cavity of the subject and a composite particle attached to the sheet portion,
wherein the composite particle containing one or more titanium oxide particles, one or more metal particles, and one or more calcium phosphate particles.

12. The medical device according to claim 11, wherein in the composite particle, the one or more titanium oxide particles, the one or more metal particles, and the one or more calcium phosphate particles are arranged in a three-dimensionally and randomly.

13. The medical device according to claim 11 or 12, wherein at least one metal particle of the one or more metal particles is fixedly attached to at least one titanium oxide particle of the one or more titanium oxide particles.

14. The medical device according to any one of claims 11 to 13, wherein the one or more metal particles are selected from the group consisting of a silver particle, a gold particle, a platinum particle, and a copper particle.

15. The medical device according to any one of claims 11 to 14, wherein the one or more metal particles comprise a silver particle and the one or more calcium phosphate particles comprise a hydroxyapatite particle.

16. The medical device according to any one of claims 11 to 15, wherein the sheet portion is breathable.

17. The medical device according to any one of claims 11 to 16, wherein the composite particle is detachably attached to the sheet portion.
